# EUROPEAN PATENT SPECIFICATION

(11) **EP 3 368 035 B1**
(45) Date of publication and mention of the grant of the patent: **23.12.2020**
(21) Application number: 16787728.1
(22) Date of filing: 21.10.2016
(51) Int. Cl.: A61K 31/426, A61K 45/00, A61K 31/496, A61P 25/00, A61P 25/18, A61P 25/22, A61P 25/28, A61P 3/04

(54) **LPA LEVEL REDUCTION FOR TREATING CENTRAL NERVOUS SYSTEM DISORDERS**
REDUZIERUNG DES LPA-SPIEGELS ZUR BEHANDLUNG VON ERKRANKUNGEN DES ZENTRALEN NERVENSYSTEMS
RÉDUCTION DU TAUX DE LPA POUR TRAITER DES TROUBLES DU SYSTÈME NERVEUX CENTRAL

(30) Priority: 30.10.2015 EP 15192351
(43) Date of publication of application: 05.09.2018
(73) Proprietor: Westfälische Wilhelms-Universität Münster, 48149 Münster (DE)
(72) Inventor: NITSCH, Robert, 55126 Mainz (DE); RADYUSHKIN, Konstantin, 55128 Mainz (DE); VOGT, Johannes, 55131 Mainz (DE)
(74) Representative: Schiweck Weinzierl Koch Patentanwälte Partnerschaft mbB
(86) International application number: PCT/EP2016/001750
(87) International publication number: WO 2017/071799

(56) References cited:
- WO-A1-2013/070879
- WO-A2-2008/051610
- US-B1- 9 051 320
- YUNG YUN C ET AL: "Lysophosphatidic Acid Signaling in the Nervous System", NEURON, vol. 85, no. 4, 18 February 2015 (2015-02-18), pages 669-682, XP029198990, ISSN: 0896-6273, DOI: 10.1016/J.NEURON.2015.01.009
- LIN M E ET AL: "Lysophosphatidic acid (LPA) receptors: Signaling properties and disease relevance", PROSTAGLANDINS AND OTHER LIPID MEDIATORS, ELSEVIER, US, vol. 91, no. 3-4, 1 April 2010 (2010-04-01), pages 130-138, XP026980791, ISSN: 1098-8823 [retrieved on 2009-03-04]
- JAMES GIERSE ET AL: "A novel autotaxin inhibitor reduces lysophosphatidic acid levels in plasma and the site of inflammation", JOURNAL OF PHARMACOLOGY AND EXPERIMENTAL THERAPEUTICS, AMERICAN SOCIETY FOR PHARMACOLOGY AND EXPERIMENTAL THERAPEUTICS, US, vol. 334, no. 1, 1 July 2010 (2010-07-01), pages 310-317, XP002666664, ISSN: 0022-3565, DOI: 10.1124/JPET.110.165845 [retrieved on 2010-04-14] cited in the application
- H. M. H. G. ALBERS ET AL: "Boronic acid-based inhibitor of autotaxin reveals rapid turnover of LPA in the circulation", PROCEEDINGS OF THE NATIONAL ACADEMY OF SCIENCES, vol. 107, no. 16, 1 April 2010 (2010-04-01), pages 7257-7262, XP055335080, US ISSN: 0027-8424, DOI: 10.1073/pnas.1001529107 cited in the application
- SELIME ÇELIK ET AL: "Correlation of binge eating disorder with level of depression and glycemic control in type 2 diabetes mellitus patients", GENERAL HOSPITAL PSYCHIATRY., vol. 37, no. 2, 1 March 2015 (2015-03-01), pages 116-119, XP055335716, US ISSN: 0163-8343, DOI: 10.1016/j.genhosppsych.2014.11.012
- RANCOULE CHLOÉ ET AL: "Involvement of autotaxin/lysophosphatidic acid signaling in obesity and impaired glucose homeostasis.", BIOCHIMIE JAN 2014, vol. 96, January 2014 (2014-01), pages 140-143, ISSN: 1638-6183
- Masaki Ino ET AL: "Alterations of Plasma Levels of Lysophosphatidic Acid in Response to Fasting of Rats", BIOLOGICAL & PHARMACEUTICAL BULLETIN (OF JAPAN), vol. 35, no. 11, 1 January 2012 (2012-01-01), pages 2059-2063, XP055572088, JP ISSN: 0918-6158, DOI: 10.1248/bpb.b12-00497

## Description

The invention relates to methods of treating central nervous system disorders, in particular neurological disorders and psychiatric disorders in a subject, comprising reducing the level of LPA in the brain of said subject.

### Background of the invention

The brain, i.e. the central nervous system, is by far the most complex organ of the human body. Due to its complexity the brain is susceptible to a variety of illnesses, such as psychiatric, neurological and neurodegenerative diseases. These diseases include, but are not limited to, schizophrenia, depression, anxiety disorders, susceptibility to stress, panic disorders, bipolar disorder, Attention Deficit Hyperactivity Disorder (ADHD), eating disorders, but also multiple sclerosis, epilepsy, Alzheimer's disease and ischemic stroke.

As the functioning of the brain still today is only poorly understood, the treatment of its illnesses still presents a significant unmet medical need.

In particular schizophrenia, depression and bipolar disorder are serious mental illnesses affecting about one in every hundred people in the western world and causes significant personal and familial suffering, as well as incurring societal and economic costs. The neurobiological causes of such illnesses can be traced to dysfunctions in synaptic transmission in the brain, and antipsychotics, which primarily rely on altering dopamine or serotonin signaling pathways, are currently recommended for treatment. However, these therapies cause frequent and sometimes serious side effects.

Recently, bioactive lipid signaling and protein-lipid interactions have been found to be involved in all steps of synaptic signaling processes. A genetic controller for this pathway, the phosphatase-like molecule "plasticity related gene 1" (PRG-1), which when "deactivated" causes behavioral deficits in mice that are indicative of mental illness, has been identified (Trimbuch et al., 2009). The genetic sequence of PRG-1 is highly conserved from laboratory animals to humans.

### Description of the invention

With the goal to more specifically characterize the role of this pathway in central nervous system disorders, the instant inventors found out that the recently reported genetic mutation (single nucleotide polymorphism (SNP)) R345T in PRG-1 interferes with the pathway through a loss of control over LPA synaptic levels and a subsequent increase in glutamate release at the synapse. This genetic mutation has a frequency of about 0.6%, corresponding to approximately 3.5 million European and 1.5 million US citizens. Further, the inventors assessed a human-population cohort using sensory and EEG measurements, and found that carriers of this genetic mutation exhibited reduced sensory gating - the ability to filter out irrelevant sensory information. Reduced sensory gating is associated with schizophrenia and other behavioral disorders.

Neurons in the human brain can be broadly divided into two classes: excitatory or inhibitory, depending on whether they tend to induce or suppress the generation of an action potential. Maintaining the correct balance of excitation and inhibition (E/I balance) ensures that neural activity is homeostatically regulated and stays in a narrow and safe range. Excitatory neurons are characterized by the release of the neurotransmitter glutamate and increased levels at the synapse lead to imbalances in the E/I systems. Dysfunctions related to this homeostatic system have been suggested to contribute to the pathophysiology of mental disorders (Harrison and Weinberger, 2005; Javitt et al., 2008). PRG-1 tightly controls the synthesis of lysophosphatidic acid (LPA), which in turn controls glutamate release at the synapse (Trimbuch et al,. 2009; YUNG YUN C et al.; "Lysophosphatidic Acid Signaling in the Nervous System", NEURON, vol. 85, no. 4 , pages 669-682 and LIN M et al.; "Lysophosphatidic acid (LPA) receptors: Signaling properties and disease relevance", PROSTAGLANDINS AND OTHER LIPID MEDIATORS,vol. 91, no. 3-4, 2010, pages 130-138).

Reinstating a normal E/I balance could provide a potential treatment for a range of mental and neurological illnesses.

In this newly identified pathway, surprisingly it has been found that the unregulated synthesis of LPA or uncontrolled synaptic LPA-levels lead to excessive glutamate release at the synapse and to a shift in the E/I balance. Therefore, reducing the lysophosphatidic acid (LPA) level in the brain may treat or prevent many central nervous system disorders.

It is therefore an aspect of the present invention to provide an inhibitor of autotaxin (AT) for use in treating or preventing psychiatric disorders in a subject, comprising reducing the level of LPA, which leads to a better regulation of glutamate release at the synapse, and thus the reinstation of the E/I balance.

As it has been found by the inventors, deactivated PRG-1 leads to uncontrolled, i.e. excessive LPA levels. Therefore, in one embodiment, the central nervous system disorders are treated by activating or upregulating PRG-1.

LPA is synthesized by the protein autotaxin which acts upstream of the LPA-LPA2/PRG-1 axis (Moolenaar & Perrakis, 2011). As it has been found by the inventors, the inhibition of autotaxin diminishes LPA levels.

Therefore, the psychiatric disorders are treated by inhibiting autotaxin.

In a preferred embodiment, the autotaxin inhibitor is 4-[3-(2,3-Dihydro-2-oxo-6-benzoxazolyl)-3-oxopropyl]-1-piperazinecarboxylic acid (3,5-dichlorophenyl)methyl ester (PF8380), or (Z)-3-((4-((3-(4-Fluorobenzyl)-2,4-dioxo-1,3-thiazolan-5-yliden)-methyl)phenoxy) methyl)benzeneboronic acid (HA130).

By choosing two structurally unrelated autotaxin inhibitors, the inventors have shown that the pharmacological effect observed is based on autotaxin inhibition, and not on any other property intrinsic to the molecules tested.

Autotaxin inhibitors are described in, i.a. WO2009046841, WO2010115491 or WO2011044978. In particular, WO2009046841 discloses a whole class of piperidine and piperazine derivatives as autotaxin inhibitors which also include PF8380.

Moreover, the inventors have shown that mice, which have an altered excitation/inhibition balance , known to be an endophenotype of psychiatric disorders related to schizophrenia in man (e.g. Harrison and Weinberger, 2005; Javitt et al., 2008), after treatment with an autotaxin inhibitor showed full recovery from altered prepulse inhibition (PPI), the best established mouse-phenotype related to psychiatric disorders such as schizophrenia (Davis, 1984; Swerdlow et al., 1994; Braff et al., 2001). This test assay is also used similarly in humans and assesses the capacity of the brain for sensory gating, found to be altered in individuals carrying a loss-of-function mutation in the PRG-1 gene, thus strongly indicating that autotaxin inhibitors may also be efficacious in humans.

Remarkably, the inventors were also able to demonstrate in a mouse model that autotaxin inhibitors effectively reduce food intake after food deprivation, indicating that they may be used to treat eating disorders, or disorders that benefit from reduced food intake, such as obesity.

There is also evidence that suggests that this pathway plays a role in other psychiatric disorders, such as anxiety disorders and ADHD as well as in neurological disorders such as multiple sclerosis and ischemic stroke. For the latter, the inventors have already collected first data in animal models showing that infarct size and stroke-related motor deficits are significantly reduced by use of the ATX-inhibiting agent PF8380.

E/I balance has been suggested to be involved in a variety of psychiatric disorders, including schizophrenia and bipolar disorder, but also panic disorders, ADHD, and more generally in resilience - the body's innate resistance to stress and adversity. This therapeutic strategy may not only be suitable for the treatment of patients suffering from mental illness, but may be also used preventively in individuals that are particularly susceptible to mental health problems.

It is also thought that LPA regulation and via this pathway regulation of neuronal hyperexcitability plays an important role in many neurological conditions such as epilepsy, multiple sclerosis, Alzheimer's disease as well as stroke. For stroke, the inventors have already gathered preliminary results that infarct size and stroke-related motor deficits are significantly reduced in a mouse model when PF8380 is administered.

Therefore the inhibitor of autotaxin (AT) is for use in the prevention or treatment of psychiatric disorders, such as schizophrenia, depression, anxiety disorders, susceptibility to stress and stress-related disorders, panic disorders, bipolar disorder, ADHD, or eating disorders, such as binge-eating disorder. Further disclosed herein is a method which relates to the treatment of neurological disorders such as multiple sclerosis, epilepsy, Alzheimer's disease and ischemic stroke. Furthermore, in another embodiment the inhibitor of autotaxin (AT) is for use in the treatment of obesity.

In accordance with the present invention, the inhibitor of autotaxin (AT) is for use in the prevention or treatment of psychiatric disorders, such as schizophrenia, depression, anxiety disorders, susceptibility to stress and stress-related disorders, panic disorders, bipolar disorder, ADHD, eating disorders, such as binge-eating disorder, or obesity..

In another aspect, the invention relates to an inhibitor of autotaxin (AT) (hereinafter referred to as "agent") capable of reducing the level of lysophosphatidic acid (LPA) in the brain of a subject, for use in the prevention or treatment of a psychiatric disorder of a subject.

The psychiatric disorder is, preferably selected from the group consisting of schizophrenia, depression, anxiety disorders, susceptibility to stress, panic disorders, bipolar disorder, eating disorders, such as binge-eating disorder and ADHD.

It is further disclosed herein that a central nervous system disorder, which is a neurological disorder may be treated, preferably selected from the group consisting of multiple sclerosis, epilepsy, Alzheimer's disease and ischemic stroke.

In an embodiment the agent is capable of reducing excitation in the brain in states of hyperexcitation related to mental illness, by the reduction of the LPA level.

In an embodiment the agent is capable of inhibiting or downregulating autotaxin, thereby achieving a reduction of LPA levels.

The agent is an inhibitor of autotaxin, preferably 4-[3-(2,3-Dihydro-2-oxo-6-benzoxazolyl)-3-oxopropyl]-1-piperazinecarboxylic acid (3,5-dichlorophenyl)methyl ester (PF8380), or (Z)-3-((4-((3-(4-Fluorobenzyl)-2,4-dioxo-1,3-thiazolan-5-yliden)-methyl)phenoxy) methyl)benzeneboronic acid (HA130).

It is further disclosed herein a pharmaceutically active substance (referred to as "agent") capable of reducing the level of lysophosphatidic acid (LPA) in the brain of a subject, which agent preferably is an autotaxin inhibitor, for the manufacture of a medicament for the treatment of obesity in the subject.

It is further disclosed herein a pharmaceutically active substance (referred to as "agent") capable of reducing the level of lysophosphatidic acid (LPA) in the brain of a subject, which agent preferably is an autotaxin inhibitor, for the manufacture of a medicament for the treatment of a central nervous system disorder, or obesity in the subject.

As used herein "treating" or "treatment" of a state, disorder or condition includes: (1) inhibiting the state, disorder or condition, i.e., arresting, reducing or delaying the development of the disease or a relapse thereof (in case of maintenance treatment) or at least one clinical or subclinical symptom thereof, or (2) relieving or attenuating the disease, i.e., causing regression of the state, disorder or condition or at least one of its clinical or subclinical symptoms.

The terms "preventing" or "prevention" refer to the prophylaxis of a condition and include preventing or delaying the appearance of clinical symptoms of the state, disorder or condition developing in a human that may be afflicted with or predisposed to the state, disorder or condition but does not yet experience or display clinical or subclinical symptoms of the state, disorder or condition.

### An animal model of central nervous system disorders

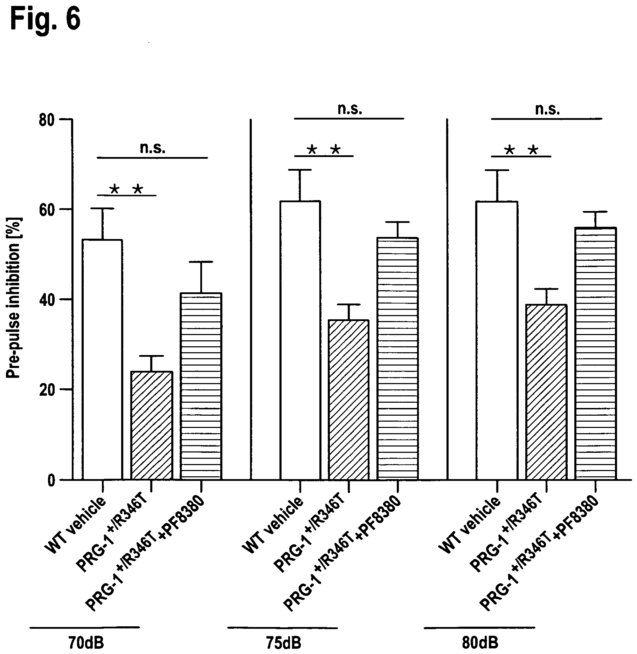

Loss of PRG-1 results in a neuronal hyperexcitation phenotype (Trimbuch et al., 2009), which is known to be present in a variety of psychiatric disorders (see Harisson and Weinberger, 2005; Javitt et al., 2008). In this regard, PRG-1 deficiency is a model for a neuronal hyperexcitation phenotype relevant for psychiatric disorders. In addition, a PRG-1 mutation is highly conserved from laboratory animals (R346T in mice) to humans (R345Tin humans), meaning that the results from the animal model should be directly translatable to humans. The inventors could show that this mutation leads to loss-of-function of PRG-1 (see below). To test this hypothesis, the inventors bred mice lacking one allele of PRG-1 (PRG-1 +/-) mimicking the situation in R345T carriers to evaluate their sensory processing capacity compared to normal wildtype mice. In vivo electrophysiological methodologies as well behavioral tests were employed to assess information processing in these mice. PRG-1 +/- mice exhibited reduced social interaction and were also less resilient to stress than wildtype mice, signs that are indicative of the behavioral traits seen in complex mental disorders. With this mouse model, which allows quantification of parameters associated with mental illness in humans, it is now possible to test prospective agents that could possibly ameliorate the symptoms brought about by this mutation. In addition to this data, the inventors have generated a knock-in mouse bearing the human mutation PRG-1R346T. Using this mouse model, the inventors have validated the PRG-1+/- mice as a model for PRG-1R346T mutation carriers (Figure 6).

### Figures

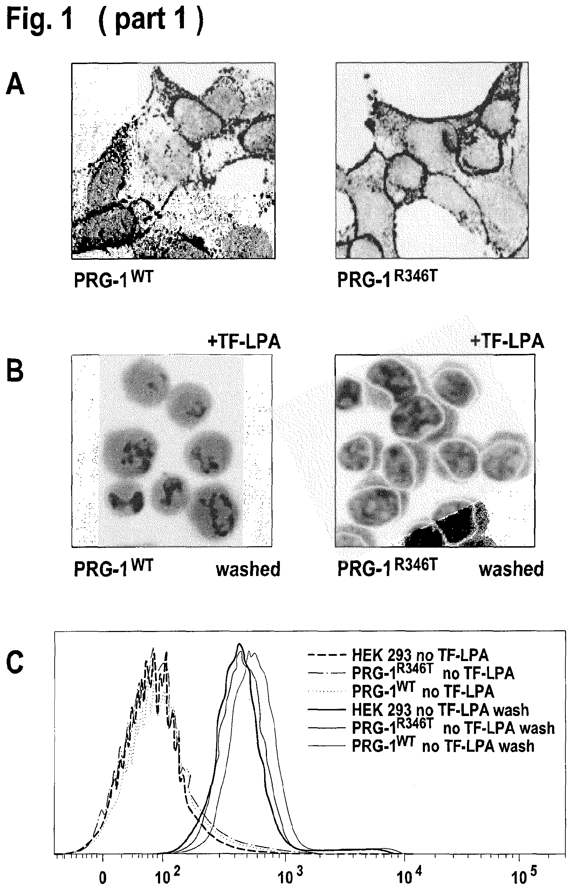

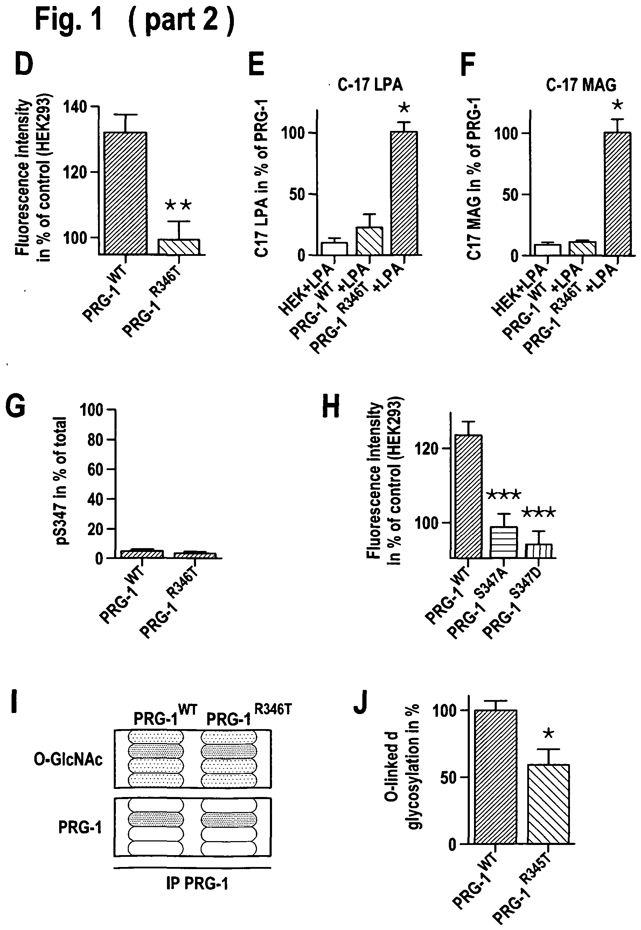

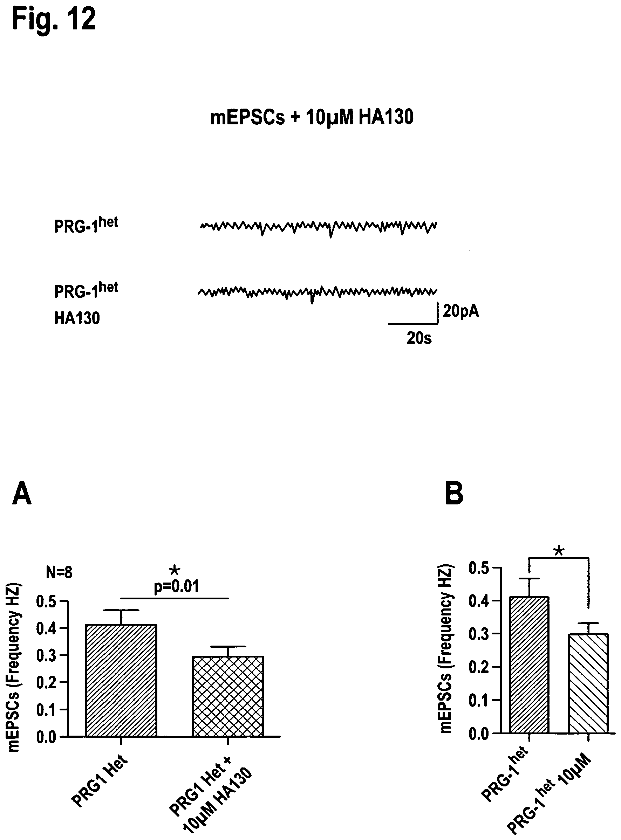

Figure 1: PRG-1R346T - loss-of-function mutation reduced LPA internalization due to altered O-glycosylation.
   **(A)** PRG-1 and PRG-1^{R346T} are expressed on HEK 293 cell membranes. **(B)** Conventional fluorescence images of transfected and TopFluor (TF)-LPA stimulated cells after washing with 0.001% SDS. Scale bar: 10 µm. **(C, D)** FACS analysis of internalized TF-LPA of PRG-1 and PRG-1^{R346T} transfected cells (n = 7 each; t-test). **(E,F)** Incubation with C-17 LPA and subsequent washing as described above, led to a significant increase of TF-LPA in PRG-1 expressing cells but not in PRG-1^{R346T} expressing cells. In line, C-17 MG, a degradation product of C-17 LPA, was significantly increased in PRG-1 expressing cells but not in control or PRG-1^{R346T} expressing cells (n = 4 each; Kruskal-Wallis test with Dunn's multiple comparisons test). **(G)** Quantitative assessment of S347 phosphorylation using isotopically labeled peptides and mass spectrometry of purified wild type PRG-1 and PRG-1^{R346T} from stable transfected HEK293 cells revealed low total phosphorylation levels for both protein variants (n = 3 measurements). **(H)** TF-LPA uptake as analyzed by FACS was significantly decreased in S347A and S347D mutant PRG-1. **(I)** Western Blot (WB) of IP using a specific PRG-1 antibody revealed decreased levels of O-GlcNAc in PRG-1^{R346T} when compared to wild type PRG-1. **(J)** Densitometric analysis of n = 7 WB from IP of PRG-1 and PRG-1^{R346T} expressing cells revealed a significantly decreased O-GlcNAc amount in PRG-1^{R346T} (t-test). All experiments were done in parallel. Bar diagrams represent mean ± S.E.M. *P<0.05,
   **P<0.01, ***P<0,001.

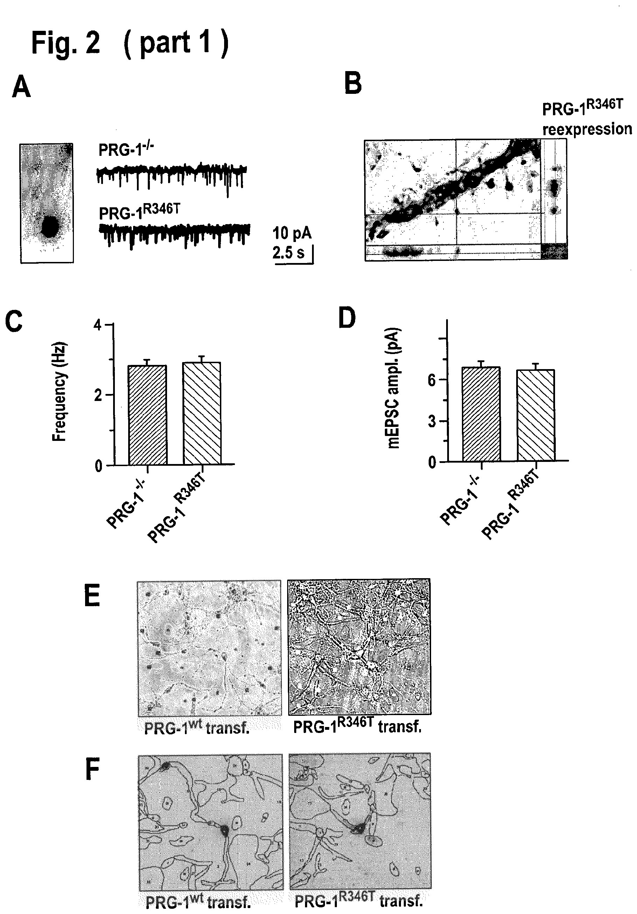

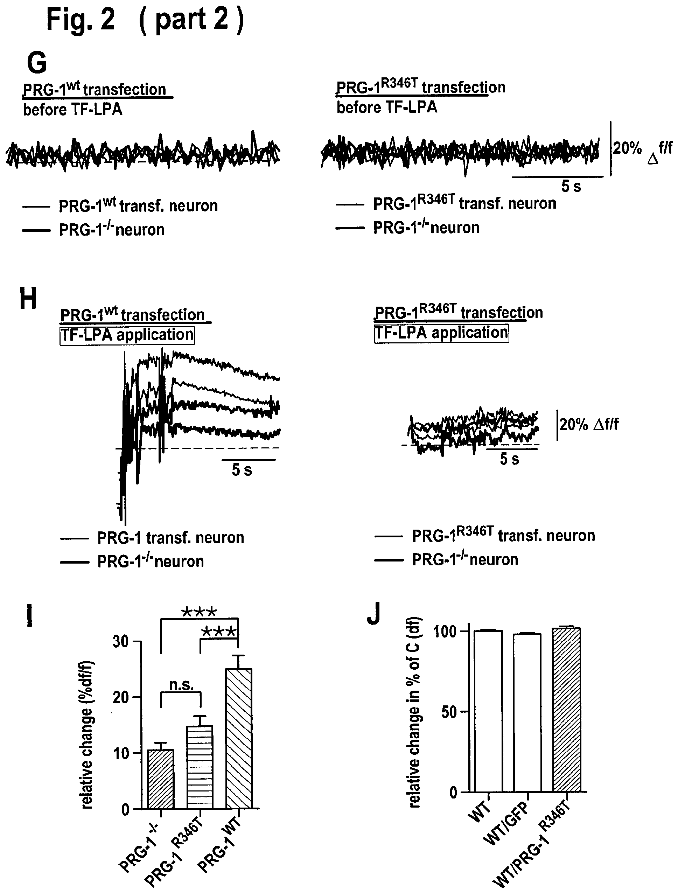

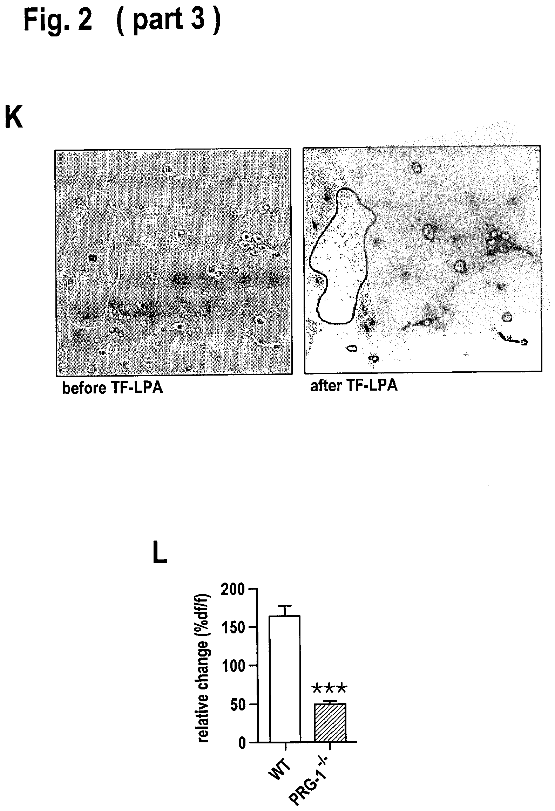

Figure 2: PRG-1^{R346T} fails to rescue PRG-1^{-/-} phenotype and to induce significant TF-LPA uptake.
   **(A, B)** In-utero electroporation (IUE) of PRG-1 and PRG-1^{R346T} leads to membrane expression of these constructs. **(C,D)** *In vitro* whole-cell patch-clamp recordings from PRG-1^{-/-} layer IV spiny stellate neurons in S1BF reconstituted either with PRG-1 **(C)** or with PRG-1^{R346T} **(D)** by IUE. (n = 11 PRG-1^{-/-} neurons and 11 PRG-1^{R346T} reconstituted neurons from 4 mice per group; *t*-test). **(E)** Bright field image and regions of interest (ROIs, yellow) of primary neuronal cultures containing PRG-1^{-/-} neurons. **(F)** Detection of transfected neurons via a cotransfected IRES-GFP cassette by fluorescent illumination. Scale bar: 50 µm **(G)** Baseline fluorescence (calculated as Δf/f) of transfected and non-transfected neurons prior to TF-LPA stimulation. **(H)** PRG-1^{wt} transfected neurons displayed a clear increase in fluorescence intensity after TF-LPA application (left), while PRG-1^{R346T} transfected neurons were not different from non-transfected PRG-1^{-/-} neurons (right). **(I)** Fluorescence intensity analysis of the ROIs revealed significant TF-LPA uptake in PRG-1^{wt} transfected neurons but not in PRG1^{R346T} transfected neurons (n = 25 neurons per condition; repeated measures ANOVA with Bonferroni post hoc correction). **(J)** Expression of GFP or of PRG-1^{R346T} in wild type neurons did not alter TF-LPA uptake when compared to non-transfected neurons (n = 57 wild type, 10 GFP transfected and 10 PRG-1^{R346T} transfected neurons; Kruskal-Wallis test with Dunn's multiple comparison test). **(K)** Bright field image and fluorescence image overview showing TF-LPA in neurons with ROIs (yellow) of a primary neuronal culture. **(L)** Fluorescence analysis revealed a significant lower TF-LPA uptake in PRG-1^{+/-} neurons when compared to wild-type neurons (n =15 neurons per group; t-test). ***P<0.001. Bar diagrams represent mean ± S.E.M.

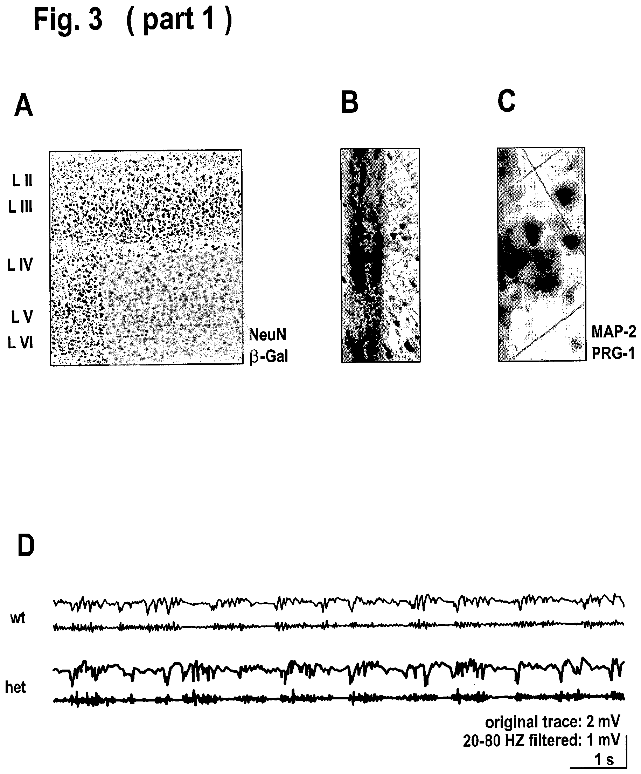

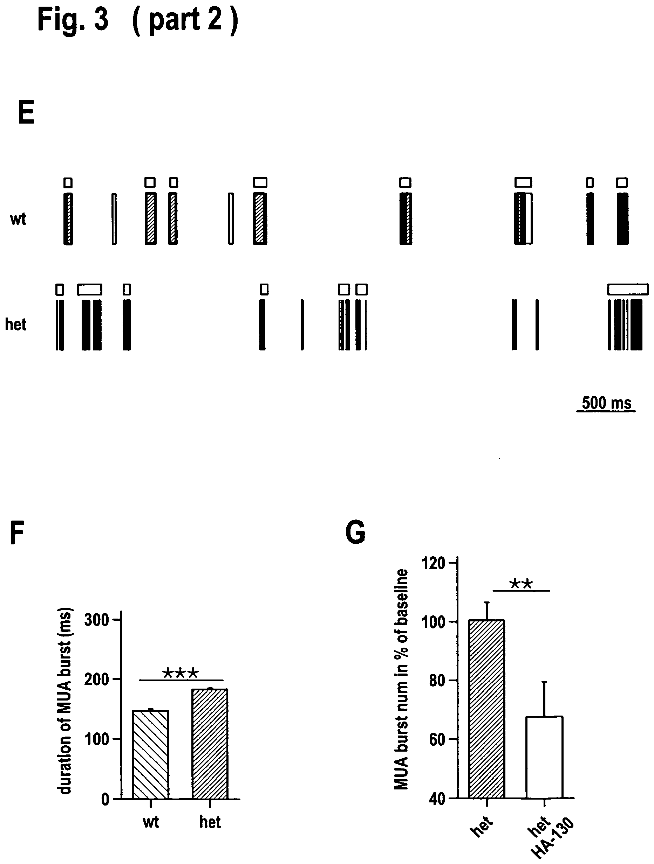

Figure 3: PRG-1 expression in the somatosensory barrel field cortex (S1BF) and in-vivo electrophysiological assessment.
   **(A)** PRG-1 is strongly expressed in layer IV neurons of the S1BF as shown by the β-Gal reporter. Scale bar: 100µm **(B)** PRG-1 is localized on dendrites and **(C)** on MAP-2-positive spines. Scale bar: 5µm **(D-F)** Cortical network assessment in PRG-1^{+/-} mice as revealed by spontaneous MUA activity (spikes: lines; bursts: bars) analyzed in wt (11379 bursts; 5 mice) and PRG-1^{+/-} (17919 bursts; 8 mice). **(G)** MUA burst numbers in PRG-1^{+/-} mice before and after application of the ATX-inhibitor HA-130.

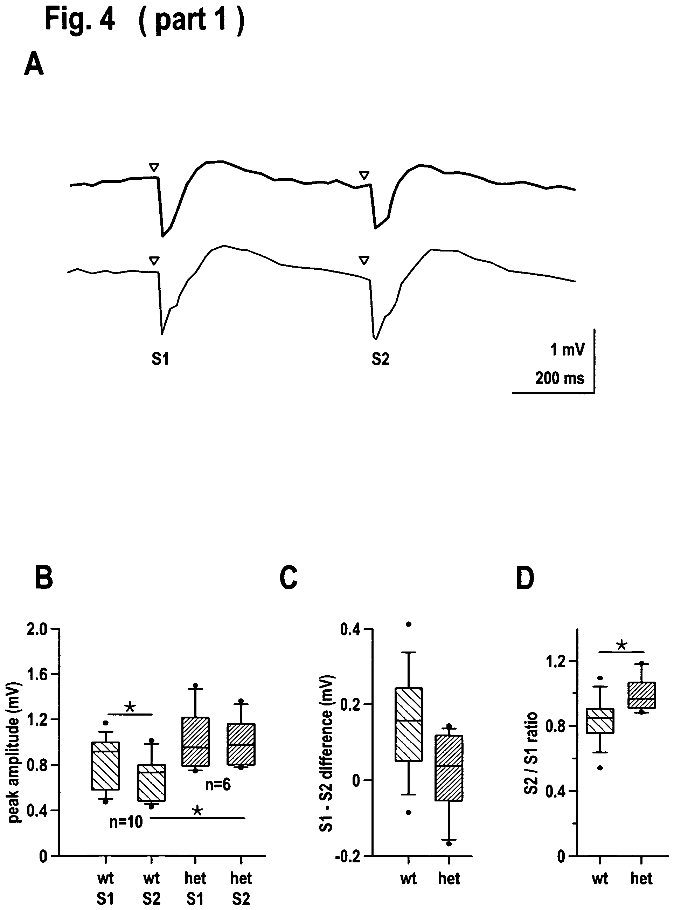

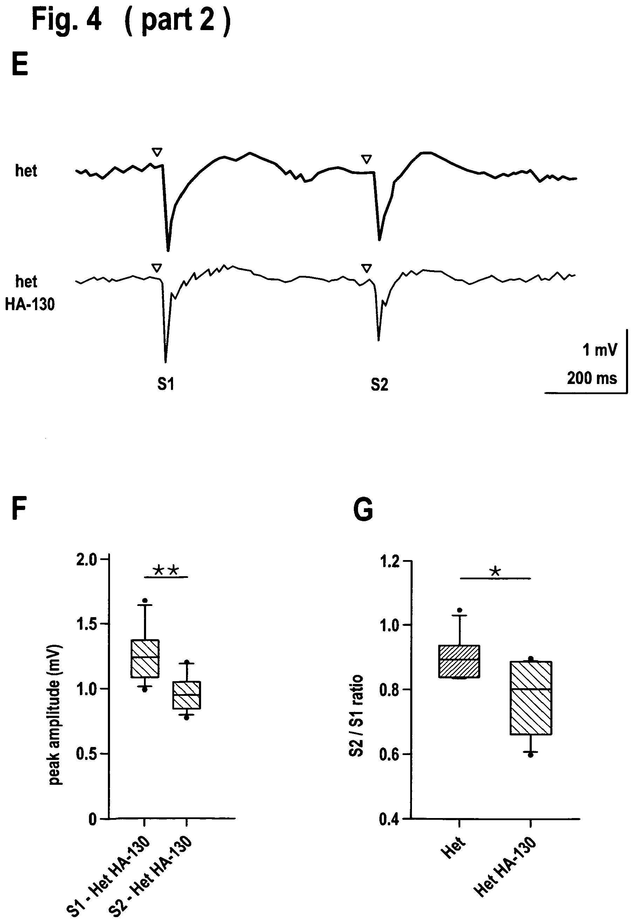

Figure 4: PRG-1^{+/-} mice display an altered sensory gating which is rescued by ATX-inhibition.
   **(A)** Paired-pulse responses (500 ms ISI) to single whisker mechanical stimulation (trace show average of 30 responses) and **(B-D)** analysis of peak amplitudes **(B),** S1-S2 difference **(C)** and S2/S1 ratio **(D,** all Mann-Whitney U-test; n = 10 WT animals and 6 PRG-1 het animals). **(E)** Paired-pulse responses before and after application of the ATX-inhibitor HA-130. **(F,G)** Peak amplitudes and S2/S1-ratio before and after ATX-inhibition by HA-130 (n = 6 animals). Data comparing paired-pulse responses before and after application of HA-130 were assessed by an one-tailed paired t-test. Box plots represent the 25^{th} and 75^{th} percentile. The middle line shows the 50^{th} percentile (median). Whiskers indicate the 10th and 90th percentiles.

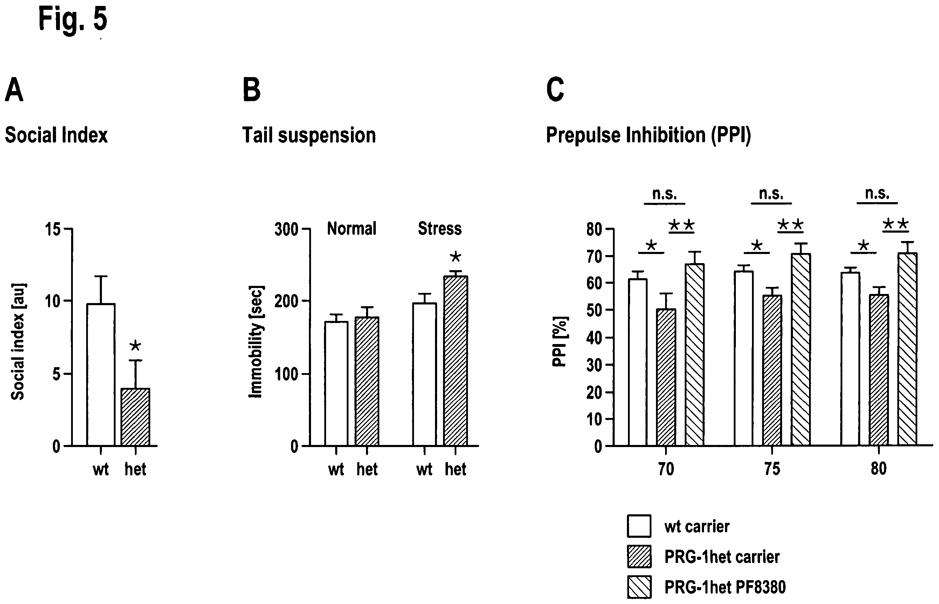

Figure 5: PRG-1^{+/-} mice display an endophenotype typical for psychiatric disorders which was rescued by ATX-inhibition.
   **(A)** The social interaction index was significantly reduced in PRG-1^{+/-} (het) mice (n = 12 wild type and 14 het mice; unpaired t-test.). **(B)** Tail suspension test under normal conditions and after exposure to acute restrain stress. (n = 14 PRG-1 het and 12 wt animals; unpaired t-test). **(C)** PRG-1^{+/-} mice displayed a robust deficit in PPI at all pre-pulse stimuli. ATX-inhibition via PF8380 treatment significantly rescued the PPI-deficit in PRG-1^{+/-} mice to a level which was not different to the PPI measured in wt mice (2way ANOVA with Bonferroni post hoc; n = 19 wt animals, 12 PRG-1 het animals and 10 PRG-1 het animals + PF8380). *P<0.05, **P<0.01. Bar diagrams represent mean ± S.E.M.
Figure 6: ATX-inhibition supresses an endophenotype of pschiatric disorders induced by a human PRG-1 mutation (PRG-1^{R346T}).
   Mice carrying a monoallelic human PRG-1 mutation (PRG-1^{R346T}) display a significantly decreased Pre Pulse Inhibition (PPI). However, ATX inhibition as shown by PF8380 treatment was able to rescue the decreased PPI to WT values. (one way ANOVA with Bonferroni post hoc; n = 9 wt animals, 15 PRG-1^{R346T+/-} animals and 15 PRG-1^{R346T+/-} animals + PF8380)**P<0.01, ***P<0.001. Bars represent mean ± S.E.M.

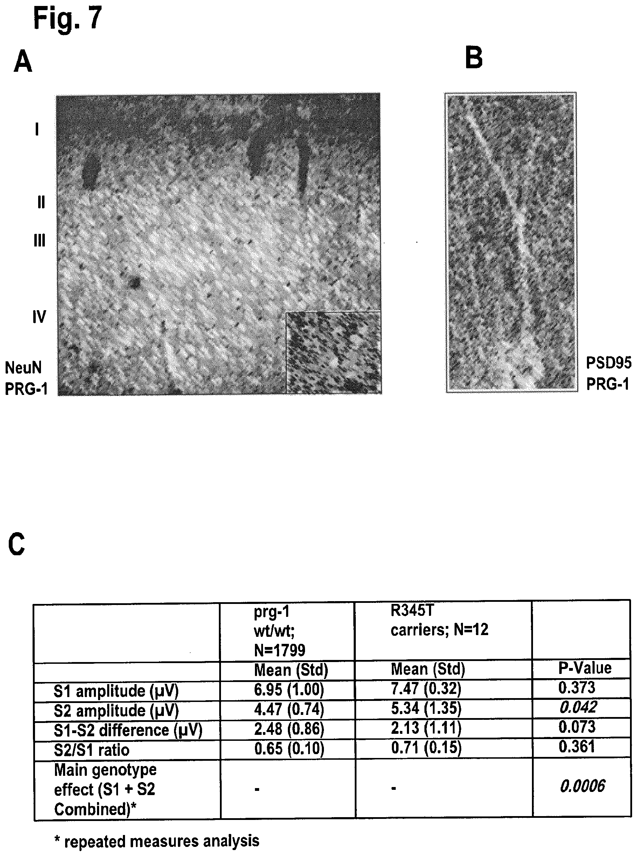

Figure 7: PRG-1 R345T: a loss-of-function human mutation induces an endophenotype for mental disorders.
   **(A)** PRG-1 is expressed in NeuN positive neurons of the somatosensory human cortex. Remark the PRG-1 expression delineating dendritic profiles of NeuN positive neurons. Insert depicts dendritic PRG-1 expression in a layer IV spiny stellate neuron at higher magnification. Scale bar: 100µm. **(B)** PRG-1 and PSD-95 punctae delineate dendritic profiles of a pyramidal neuron. Higher magnification (insert) exemplarily shows PRG-1/PSD95 colocalization (arrow heads). **(C)** Stratified analysis of *prg-1* genotype on P50 event-related potential in mutPRG-1 carriers.

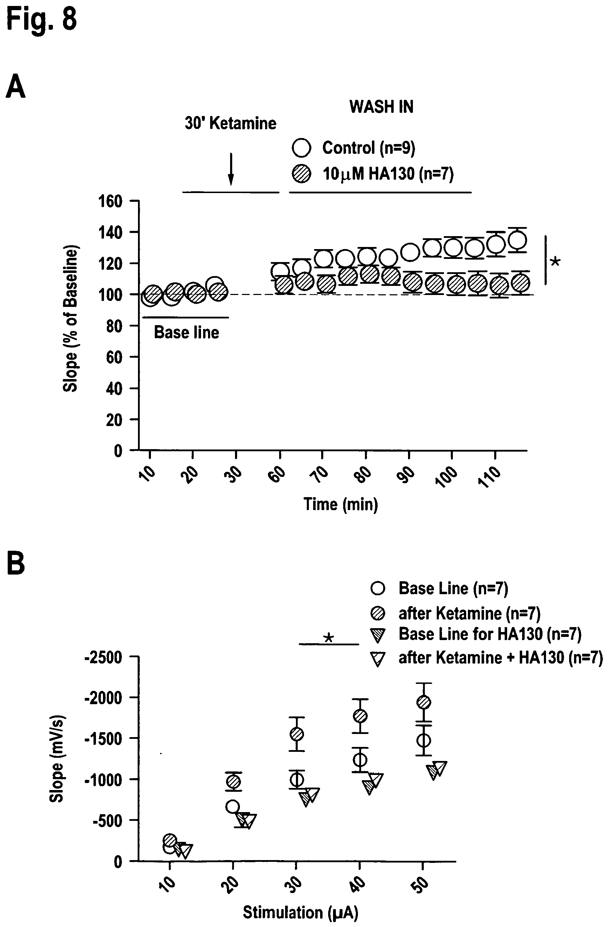

Figure 8: Neuronal hyperexcitability induced by Ketamine application was reduced by ATX-inhibition as shown by local field potential (LFPs) analyses.
   **(A)** Application of HA130 during ketamine wash out inhibited the excitatory potentiation as shown by evoked LFPs (2way ANOVA; n=9 controls and n=7 after 10µM HA130 application). Values represent mean ± S.E.M.
   **(B)** Input-output curves reflect potentiation after ketamine application (when compared to baseline before ketamine application). However, after HA-130 treatment, ketamine-induced potentiation was reverted to baseline values.

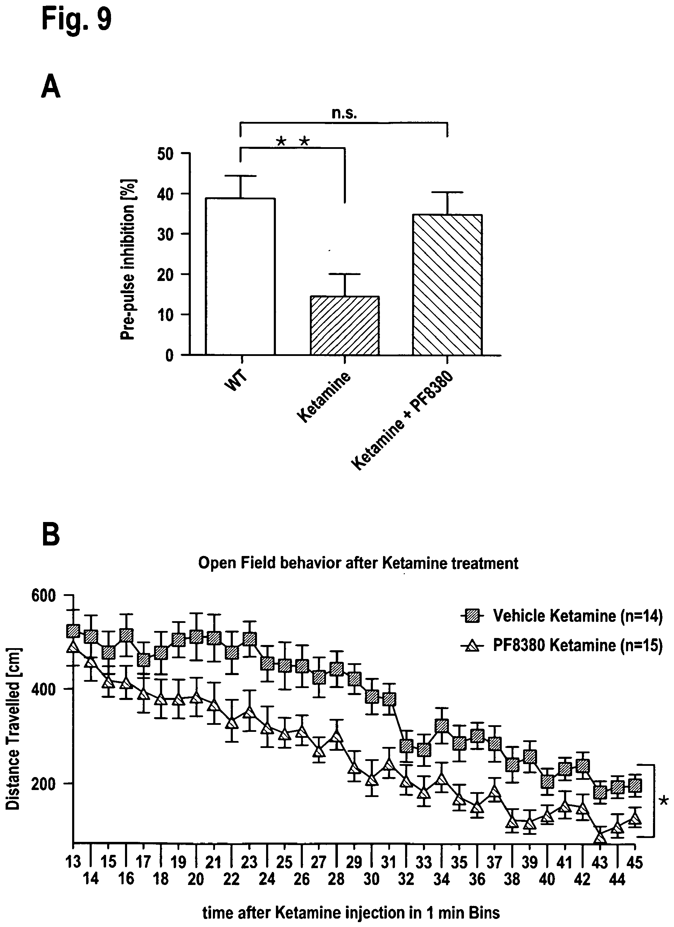

Figure 9: ATX-inhibition reverses characteristic behavior in an animal model for schizophrenia.
   **(A)** PPI analyzed in an animal model of schizophrenia induced by ketamine was significantly decreased and was rescued to WT values by ATX inhibition by PF8380.
   **(B)** Hyperactivity following ketamine application as displayed in an open field setting was significantly reduced upon ATX-inhibition by PF8380.

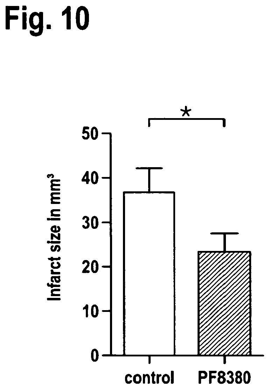

Figure 10: ATX-inhibition by PF8380 reduces the infarct size in a medial cerebral artery occlusion (MCAO) mouse model of stroke.
   **(A)** ATX-inhibition significantly reduced infarct volume measured 1day (1h MCAO) and 3 days (30 min MCAO) after MCAO.
   **(B)** ATX inhibition by PF8380significantly decreased infarct volume (30 min MCAO measured 3 d after stroke) in PRG-1+/- mice, which are an animal model for a human loss-o-function PRG-1 variant (PRG-1^{R346T}) resulting in cortical hyperactivity.

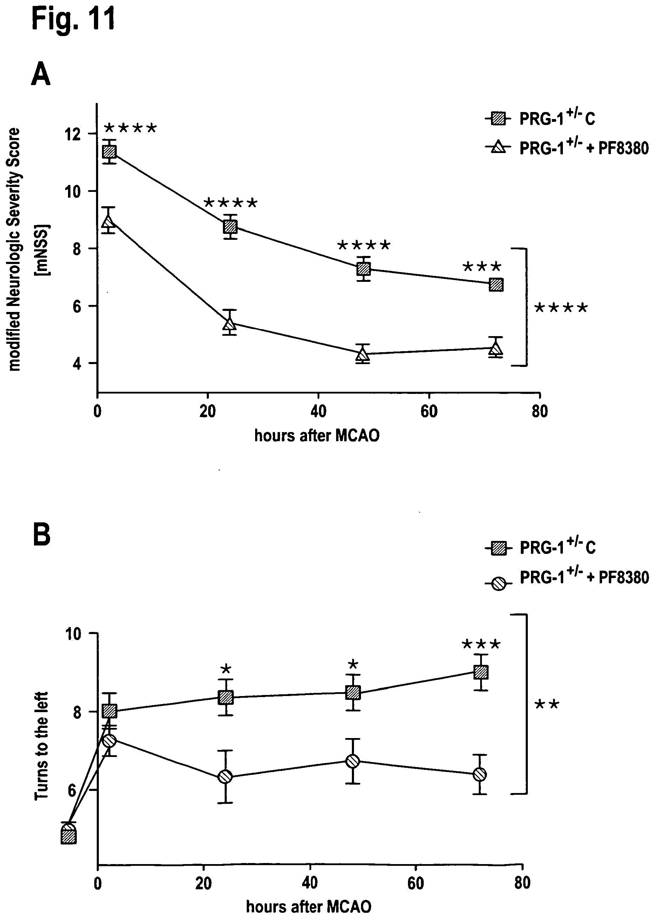

Figure 11: ATX-inhibition results in improved neurological score and a better motor performance in a medial cerebry artery occlusion (MCAO) mouse model of stroke in PRG-1^{+/-} mice, the animal model for a human loss-o-function PRG-1 variant (PRG-1 ^{R346T}) resulting in cortical hyperactivity.
   **(A)** ATX-inhibition significantly improves outcome in a modified Neurologic Severity Score (Chen J et al. 2001) after stroke in PRG-1^{+/-} mice, an animal model for cortical hyperexcitability mimicking a human PRG-1^{R345T} mutation (N=11 PRG-1^{+/-} and 13 PRG-1^{+/-}+PF8380 mice. 2-way-ANOVA, and post hoc multiple comparisons using Bonferroni adjustment for comparison at 3h, 24h, 48h and 72h post MCAO. ***p<0.001, **** p<0.0001. Diagrams represent mean ± S.E.M.
   **(B)** Long-term sensorimotor dysfunction after stroke as shown by the corner test (Zhang L et al. 2002) was significantly improved in PRG-1^{+/-} mice by ATX-inhibition N=11 PRG-1^{+/-} and 13 PRG-1^{+/-}+PF8380 mice. 2-way-ANOVA *p<0.05, ***p<0.001 significant. Multiple Comparisons following 2-way-ANOVA significant 24h post MCAO and up to 72 h post MCAO
Figure 12: HA130 decreases mEPSCs in PRG-1 het mice.
   **(A)** ATX-inhibition by 10 µM HA-130 (left) decreased mEPSCs in PRG-1 het mice suggesting a gen-dose dependent effect of PRG-1 deficiency (paired Student's t-test; n = 8).
   **(B)** ATX-inhibition by 10 µM HA-130 decreased mEPSCs which reflect the spontaneous glutamatergic release at presynaptic terminals and which are mediated via presynaptic LPA₂ receptors (Trimbuch et al., 2009) (paired Student's t-test; n = 8).

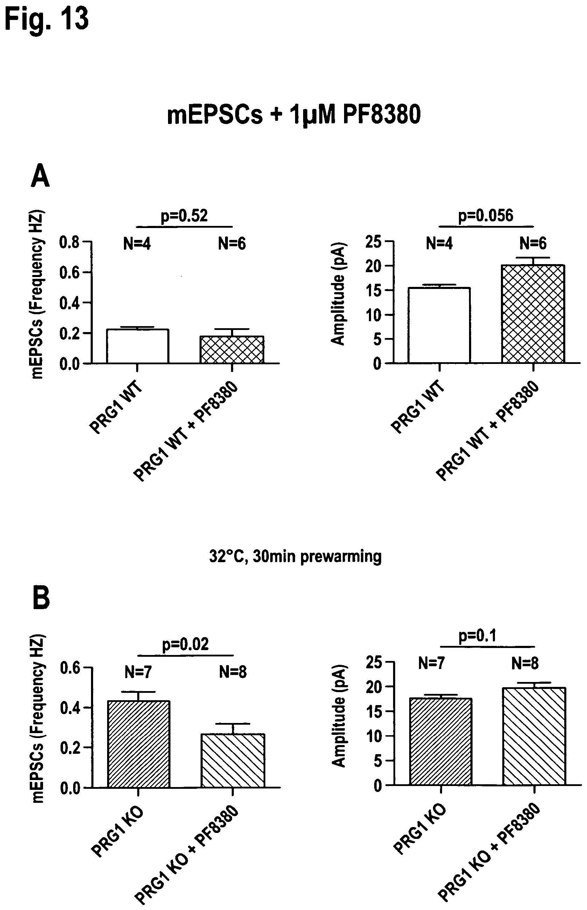

Figure 13: PF8380 does not affect mEPSC in wild type animals, but reduces mEPSC in PRG-1 Ko mice.
   **(A)** ATX-inhibition by PF8380 did not affect mEPSC frequency and amplitudes in wild type (WT) animals.
   **(B)** However, under conditions of increased synaptic phospholipid concentrations as present in PRG-1 Ko mice, application of 1µM PF8380 significantly reduced mEPSC frequency (left) and thereby neuronal hyperexcitability, while not influencing their amplitude (right, paired Student's t-test).

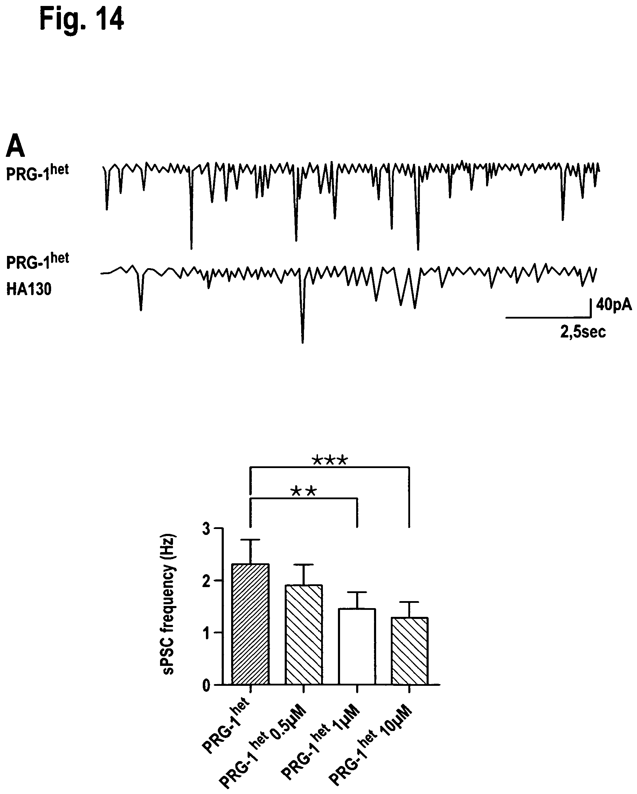

Figure 14: HA130 decreases sPSC's in PRG-1^{+/-} mice.
   ATX-inhibition in PRG-1^{+/-} mice, which display a neuronal hyperexcitability, revealed a significant decrease of spontaneous postsynaptic currents (sPSC) after treatment with 1µM and 10µM HA130. sPSCs are the sum of the overall excitatory and inhibitory input at the single neuron level and reflect the excitation/inhibition (e/i) balance.
Figure 15: PF8380 decreases eEPSC's.
   PF8380 application in hippocampal slices from PRG-1 KO mice significantly affected evoked potentials (left), but did not alter a second stimulus applied at an interval of 50 ms (middle) resulting in an increased PPR (right, all paired Student's t-test).
Figure 16: Simultaneous *in-vivo* recordings of the MEC and the hippocampal CA1 in freely moving mice show impact of PF-8380 on overexcitation phenotype.
   **(A)** Frequency of theta coherence between MEC and CA1 (7-12 Hz) and **(B)** coherence in the theta and gamma range of WT, PRG1-/- and PF8380 treated PRG1-/- mice (n = 15 WT mice, 13 untreated PRG1-/- and 7 PF8380 treated PRG1-/- mice). See also Supplementary Fig. S5. **(C)** Peak frequency of theta coherence between MEC and CA1 was lower in PRG1-/- and PF8380 treated PRG1-/- mice than in WT mice. **(D)** peak frequency of gamma coherence **(E, F)** Gamma coherence between MEC and CA1 was higher in PRG1-/- mice and was reduced to WT levels by PF8380 treatment while mean theta coherence was not affected by PRG1 deletion. One way ANOVA with Bonferroni correction was applied for normal distributed data and Kruskal-Wallis and Dunn's multiple comparison test for nonparametric data. **(G)** In line with effective concentrations of PF8380 in the cerebrospinal fluid (CSF; see Figure12), ATX-inhibition by this substance resulted in a significant decrease of the main LPA species (LPA 16:0, 18:1, 18:2), which account for most of the brain LPA content (n=6 for all groups except for LPA18:2 PRG-1+vehicle with n =5; one tailed, Mann-Whitney-U-test). To avoid bias by a potential blood contamination, which contains higher LPA-levels and may occur during CSF extraction, values exceeding mean + 2xSD were excluded.

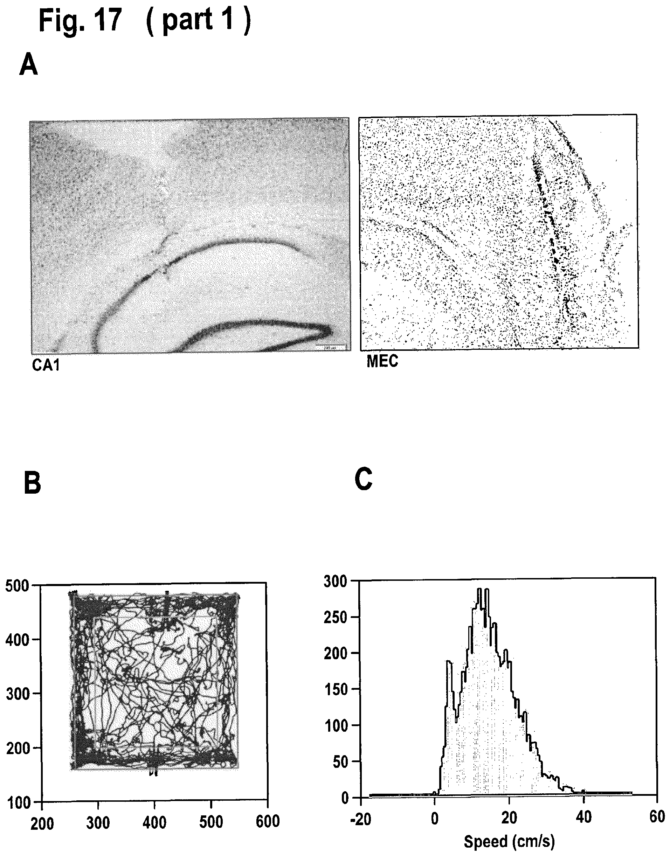

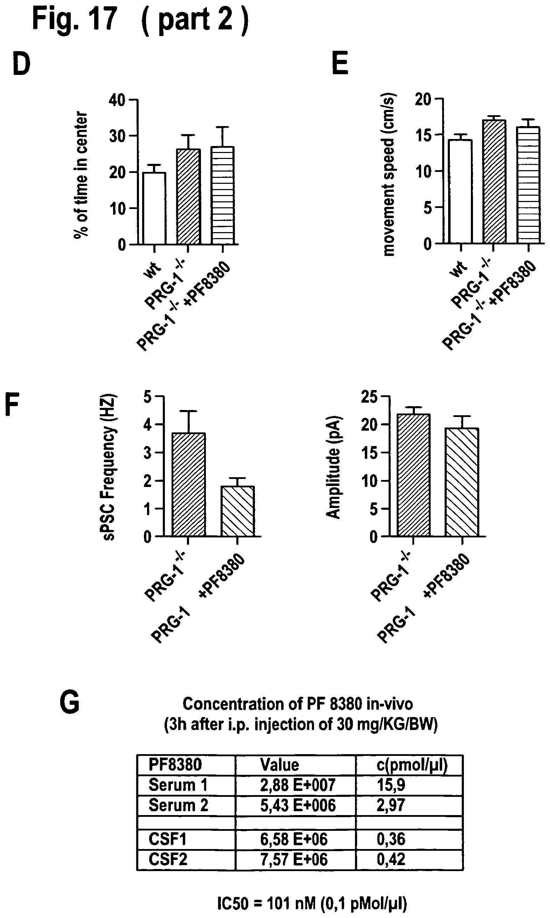

Figure 17: In-vivo recording in the layer II MEC and in the hippocampal CA1 of freely moving mice show specific action of PF 8380 on excitatory phenotype.
   **(A)** Nissl stained brain slices depict the electrode position in the hippocampal CA1 region and in the layer II of the medial entorhinal cortex (MEC). Scale bar: 200 µm.
   **(B)** example of a trace showing mouse exploratory behavior in the open field during simultaneous recording in the CA1 and the MEC layer II. **(C)** example of the speed profile of the mouse shown in b during exploratory behavior in the open field. **(D, E)** wild type (n = 15) and PRG-1-/- (untreated n = 10 and PF8380-treated n = 6) mice showed a similar preference and no difference in the average movement speed during exploratory behavior in the open field environment (one-way ANOVA). **(F)** application of 1µm PF8380 which blocks the LPA synthesis molecule Autotaxin (ATX) significantly reduced spontaneous postsynaptic currents (sPSC), which are the sum of the overall excitatory and inhibitory input, on single neuronal level while not affecting the amplitude (n = 13 untreated and 12 PF8380-treated PRG-1-/- neurons). Data was calculated using an one-tailed Mann-Whitney-test. **(G)** In-vivo application of PF8380 in therapeutic concentrations of 30 mg/KG/BW25 resulted in robust concentrations in the serum and effective levels in the cerebrospinal fluid (csf) which were up to 4 times higher than IC50.

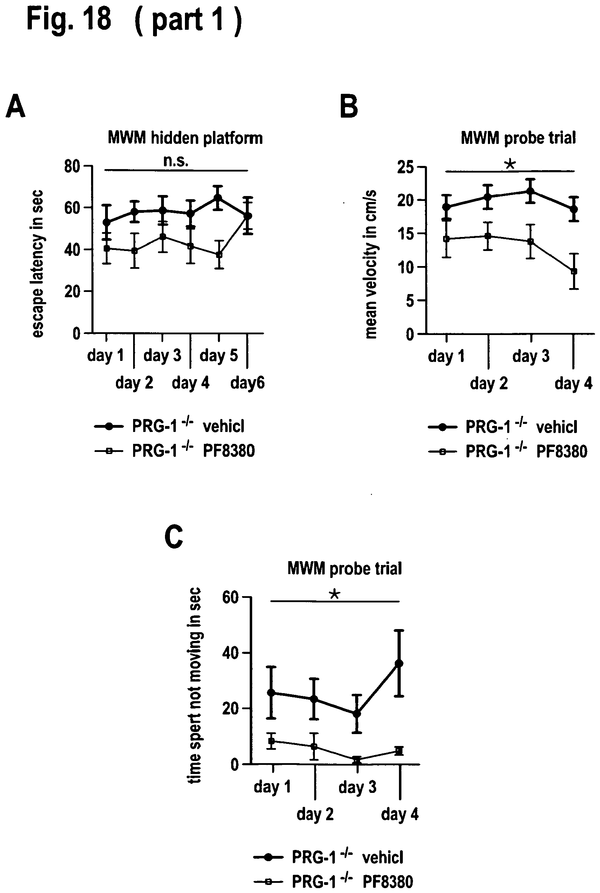

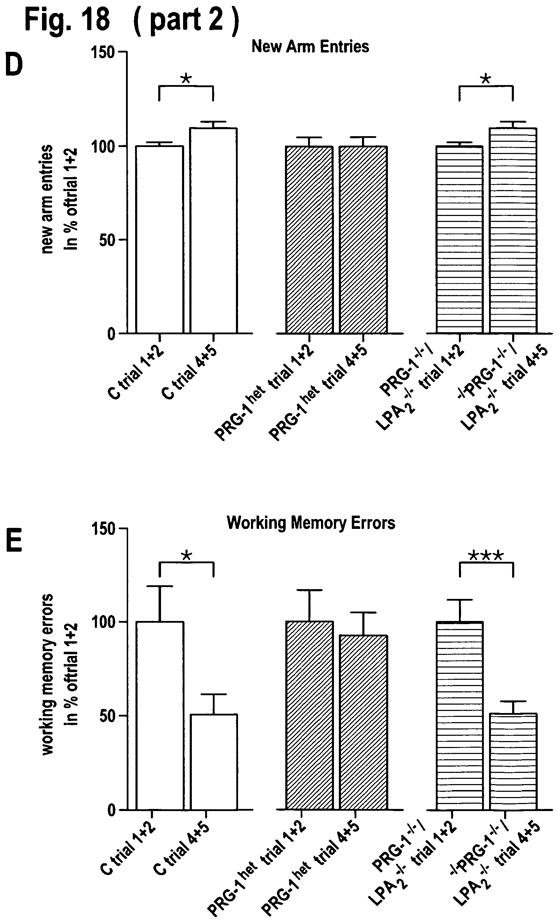

Figure 18: Assessment of spatial learning in PRG-1-/- mice show specific impact of PF 8380 on hyperexcitation-related behavioral deficits.
   **(A)** Escape latency in the Morris-Water-Maze in untreated (n = 10) and PF8380-treated (n = 7) PRG1-/- mice did not change during training days. **(B, C)** Mean movement velocity and time spent without moving (immobility) during probe trials were significant different between PF8380-treated and untreated PRG-1-/- animals. All data represent mean ± s.e.m. Data were calculated using a two-way RM ANOVA. **(_D, E)** working memory errors and new arm entries of trials 4+5 are displayed as percent of trials 1+2 (n = 10 WT, 10 PRG-1-/- and 10 PRG-1-/-/LPA2-/- animals). Data were calculated using a two-tailed paired t-test for WT and PRG1-/- mice and a one-tailed paired t-test for the LPA2-R-/-/PRG1-/- group and a Wilcoxon matched pairs signed rank test when data was not normal distributed. All data represent mean ± s.e.m. *p<0.05; ***p≤0.001.

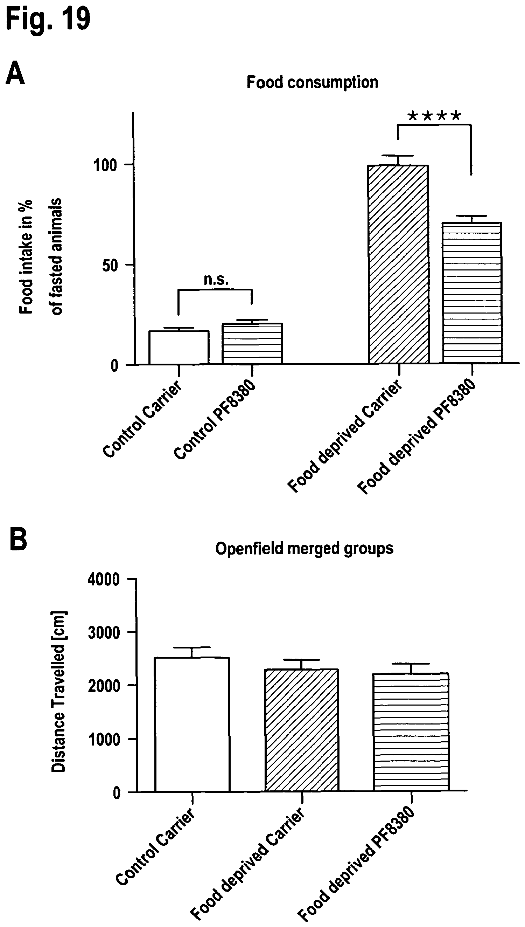

Figure 19: ATX-inhibition reduces food deprivation-induced food intake.
   **(A)** ATX-inhibition does not alter food intake in control mice. However, when overnight food deprived animals were exposed to food, PF8380-treated animals displayed a significantly decreased food intake (within 60 min) pointing to a specific cortical effect of PF8380 on obesity-related behavior (n = 31 control (C) and 31 C+PF8380, 45 food deprived and 45 food deprived + PF8380 animals, Mann-Whitney-U test ****p<0,0001).
   **(B)** Controls, over-night food deprived controls and over-night food deprived animals treated with PF8380 did not display significant differences in locomotion in the open field (OF, 10 min) behavior as assessed by total distance (n = 12 animals per group, Kruskal-Wallis test).

### Experimental Examples illustrating the Invention

### PRG-1^{R346T} is a loss-of-function mutation

PRG-1 was shown to be involved in internalization of lysophosphatidic acid (LPA) to intracellular postsynaptic compartments (Trimbuch et al., 2009), thereby controlling LPA levels in the synaptic cleft. To understand the molecular consequences of the human SNP resulting in arginine (R) to threonine (T) exchange at position 345 in the amino acid chain of PRG-1, the inventors established HEK cell lines with stable expression of the mouse homolog of this SNP, PRG-1^{R346T}, and found similar membrane localization when compared to wild type PRG-1 (wtPRG-1, Fig. 1A). After application of fluorescence labeled LPA (TopFluor(TF)LPA) and removal of surface bound TF-LPA using 0,001% SDS, PRG-1^{R346T} expressing cells displayed a lower fluorescence signal in intracellular compartments (Fig. 1B). This finding was quantified by FACS analysis (example shown in Fig. 1C) revealing a significantly reduced capacity of PRG-1^{R346T} expressing cells to internalize TF-LPA when compared to wtPRG-1 (Fig. 1D). To unambiguously prove the internalization of LPA, the inventors applied a chemical modified, unnatural LPA (C17-LPA) on wtPRG-1 expressing cells which showed a statistically significant internalization of C17-LPA (Fig. 1E) and the intracellular presence of its metabolite C17-MAG (Fig.1F) when compared to HEK-cells which lack any PRG-1 expression. In line with previous results, PRG-1^{R346T} expressing cells did not show any significant increase of intracellular C17 LPA nor of its metabolite C17-MAG providing further evidence that PRG-1^{R346T} lacks the capacity to mediate internalization of LPA into cells (Fig. 1E, F).

### PRG-1^{R346T} alters O-glycosylation, but not phosphorylation of neighboring S347

Since PRG-1^{R346T} was properly expressed at the membrane, the inventors hypothesized a posttranslational modification being involved in the loss-of-function in TF-LPA internalization.

Interestingly, the SNP changing *R345* (arginine) to 345*T* (threonine) is located in close proximity to a frequently reported phosphorylation site (Goswami et al., 2012; Huttlin et al., 2010; Munton et al., 2007; Trinidad et al, 2008; Wisniewski et al., 2010). Therefore, the inventors addressed the question to what extent the neighboring S (serine) at position 346 in humans and at position 347 in mouse is phosphorylated under wild type conditions, and whether this phosphorylation is eventually altered due to the mutation of R to T, which alters typical phosphorylation motifs (Pearson & Kemp, 1991). Using quantitative mass spectrometry analysis and isotopically labeled peptides corresponding to the peptide containing S347, the inventors detected that, although described as a potential activity-related phosphorylation site (Munton et al., 2007), S347 neighboring the SNP site showed only minor phosphorylation (4.73% of total PRG-1, Fig. 1G). In addition, in PRG-1 ^{R346T} the inventors could neither detect any relevant change in phosphorylation of S347 (3.33% of total PRG-1, Fig. 1G), nor any phosphorylation of the *T* at position 346. To further assess whether S347 phosphorylation is a potential relevant cause for the loss of LPA-uptake of PRG-1^{R346T}, the inventors established HEK cell lines with a stable expression of PRG-1^{S347A} and PRG-1^{S347D}, which either mimic the non-phosphorylated (S347A) status or the phosphorylated status (S347D) of S347. Both cell lines lacked the capacity to significantly internalize TF-LPA above baseline levels (Fig. 1H), strongly arguing against an important role for phosphorylation explaining the loss-of-function of PRG-1^{R346T} in TF-LPA internalization, however, pointing to the need of S347 at this position. Another intracellular post-translational modification, critically important for brain function is *O*GlcNAC glycosylation (Rexach et al., 2008), which occurs at S and T residues and has a complex interplay with phosphorylation (Tarrant et al., 2012). Using immunoprecipitation studies and specific antibodies, the inventors analyzed *O*-GlcNAC glycosylation in wtPRG-1 in comparison to PRG-1^{R346T} and detected significantly reduced glycosylation levels in PRG1^{R346T} expressing cells (Fig. 1I,J), indicating an interference of the R346T mutation with its neighboring S347 O-glycosylation site. Since neither *A* nor *D* are a target for *O-*GlcNAC glycosylation and the cell lines expressing PRG-1^{S347A} and PRG-1^{S347D} lacked the capacity of internalizing TF-LPA (Fig. 1H), these data point to the necessity of an O-glycosylation at *S* for proper PRG-1 function, which is altered in PRG-1^{R346T}.

In sum, these data on post-translational modifications provide evidence for the fact that not changes in phosphorylation, but proper O-glycosylation of *S* at position 346 in humans interfered by the R345T SNP is crucial for PRG-1 function.

### PRG-1^{R346T} is a loss-of-function mutation at the synapse

To analyze the potential functional consequences of the human SNP in neurons, the inventors then assessed PRG-1^{R346T} function at the synapse, and performed whole-cell patch-clamp recordings from layer IV spiny stellate neurons in the S1BF of PRG-1^{-/-} animals that reexpressed PRG-1^{R346T} delivered by *in utero* electroporation (Fig. 2A). This re-expression resulted in proper localization of PRG-1^{R346T} protein at the spinehead membrane, excluding an impact of this mutation in protein targeting (Fig. 2B). While re-expression of wtPRG-1 in PRG-1 deficient neurons was reported to rescue the frequency of miniature excitatory postsynaptic currents (mEPSC) to wild-type levels in hippocampal CA1 pyramidal cells (Trimbuch et al, 2009), PRG-1^{R346T}, albeit present at the proper dendritic localization, failed to achieve this functional rescue. This is consistent with a loss-of-function of PRG-1^{R346T}, presumably due to its inability to support LPA-internalization (Fig. 2A, C).

To directly assess the functional consequences of PRG-1^{R346T} in neurons in terms of their capacity to internalize LPA (Trimbuch et al., 2009), the inventors used primary cortical neurons obtained from PRG-1^{-/-} animals either transfected with a *prg-1*^{wt} or with a *prg-1*^{R346T} expressing construct (Fig. 2E, F) and measured internalization of LPA which was tagged by a fluorescence label (TopFluor, TF-LPA) in a live imaging mode. TF-LPA internalization capacity of either PRG-1^{wt} or PRG-1^{R346T} was compared to PRG-1 deficient neurons in the same experiment (see also Fig. 1G for baseline levels). While expression of PRG-1^{wt} in neurons induced a significant increase in TF-LPA internalization, PRG-1^{R346T} expression failed to do so (Fig 2H, I). To rule out a dominant-negative effect of PRG-1^{R346T}, the inventors used the same approach and transfected cortical neurons obtained from wild type mice either with a *prg-1*^{R346T} or a control GFP-construct and found no alteration on TF-LPA-internalization in the transfected neurons (Fig. 2J). These studies confirmed that PRG-1^{R346T} was a loss-of function mutation in a neuronal context. Finally, since the reported human SNP was only detected in monoallelic carriers, the inventors analyzed the impact of a monoallelic functional loss in cortical neurons obtained from PRG-1^{+/-} animals and found that already monoallelic PRG-1 loss was sufficient to significantly decrease TF-LPA-internalization when compared to wildtype neurons (Fig 2K, L).

### Altered phospholipid modulation due to monoallelic loss of synaptic PRG-1 affects cortical E/I balance

In order to characterize the role of PRG-1 in cortical networks, the inventors then assessed expression of PRG-1 in the cerebral cortex and found strong expression in layer IV of the mouse somatosensory barrel field cortex (Fig 3A) which was restricted to glutamatergic neurons. Analysis of subcellular localization revealed that PRG-1 was expressed at dendritic spines (Fig. 3B,C). Since heterozygous deletion of PRG-1, leaving only one functional *prg-1* allele, results in a linear reduction of protein expression of approximately 50% (Trimbuch et a.l, 2009), and heterozygous PRG-1-deficiency significantly reduced the functional capacity of PRG-1^{+/-} neurons to internalize TF-LPA in a similar range (Fig. 2K, L), PRG-1^{+/-} animals are to be assumed as an animal correlate of human monoallelic PRG1^{R345T} carriers who only have one functional *prg-1* allele not affected by the mutation. To understand implications of such a reduced synaptic PRG-1 function in intracortical information processing, the inventors performed multi-channel extracellular recordings in the S1BF cortex of wt and PRG-1^{+/-} mice *in vivo* (Fig. 3D). Spontaneous activity in PRG-1^{+/-} mice showed a significant prolongation of multiunit activity (MUA) burst duration when compared to wt mice (Fig. 3E, F). This finding points to a change in the cellular E/I balance towards excitation within cortical microcircuitries which has been related to severe behavioral deficits (Yizhar et al., 2011).

### Inhibition of LPA-synthesis reverts altered cortical information processing in monoallelic PRG-1 deficient mice

To test for involvement of phospholipids at the cortical network level, the inventors analyzed the effect of inhibiting the LPA-synthesizing molecule autotaxin (ATX) which acts upstream of the LPA-LPA₂/PRG-1 axis (Moolenaar & Perrakis, 2011).

Surprisingly, HA-130, a recently described specific inhibitor of ATX (Albers et al., 2010), significantly decreased spontaneous postsynaptic currents (sPSC), which are the sum of the overall excitatory and inhibitory input reflecting the total E/I-balance (Fig. 14). In line with these findings *ex vivo,* already at concentrations as low as 1µM, HA 130, applied into the cortex of living PRG-1^{+/-} mice, significantly decreased the number of multi-unit activity (MUA) bursts (Fig. 3G).

In addition to analysis of spontaneous cortical network activity, the inventors assessed pre-attentive cortical information processing and measured sensory gating in a double-pulse whisker stimulation model (Fig 4A). Local field potentials (recorded in the whisker-specific cortical barrel) in response to repetitive single-whisker stimulation elicited larger amplitudes to second stimulus in PRG-1^{+/-} when compared to wt (S1; S2; ISI=500 ms; Fig. 4B). Larger S2amplitudes altered S1-S2 values and resulted in a statistically significant increase of the S2/S1 ratio (Fig. 4C, D). These findings indicate that already loss of one functional allele of *prg-1* as present in mutPRG-1 carriers, and thus a reduction of about 50% of functional PRG-1 at the synapse, causes an apparent E/I-imbalance in cortical networks and an altered sensory gating. To prove whether phospholipid modulation is able to directly affect cortical information processing, the inventors applied HA-130 in the double-pulse whisker stimulation model (Fig 4E). This inhibition of LPA-synthesis significantly restored sensory gating in animals with monoallelic PRG-1-deficiency as shown by reduction of S2 values (when compared to S1 values) and a reduced S2/S1-ratio (Fig. 4F, G).

### Pharmacological intervention into monoallelic PRG-1 deficiency reverts behavioral deficits characteristic for central nervous system disorders

To answer the question whether observed alterations in excitatory synaptic function and cortical network activity result in a behavioral phenotype, the inventors assessed monoallelic PRG-1 deficient animals (PRG-1^{+/-}). Using a three chambered box paradigm (Radyushkin et al., 2009), a significantly reduced social interaction index in PRG-1^{+/-} mice was found (Fig 5A). The inventors further assessed PRG-1^{+/-} mice under normal conditions and after application of environmental stress, known to be a risk factor for mental disorders (van Winkel et al., 2008). While PRG-1^{+/-} mice displayed no differences under normal conditions in tail suspension tests, following acute stress, PRG-1^{+/-} mice showed a significant higher immobility (Fig. 5B), which all together resembled behavioral aspects of complex mental disorders (Cryan et a.l, 2005; Inta et al., 2010; Shen et al., 2008).

Since a reduced social interaction and altered behavior in tail suspension test reflects symptoms of mental disorders, the inventors assessed pre-pulse inhibition (PPI) in these animals. PPI is highly conserved among vertebrates, is indicative for altered mental function, and is one of few paradigms in which humans and rodents are tested in similar fashions (Davis, 1984).

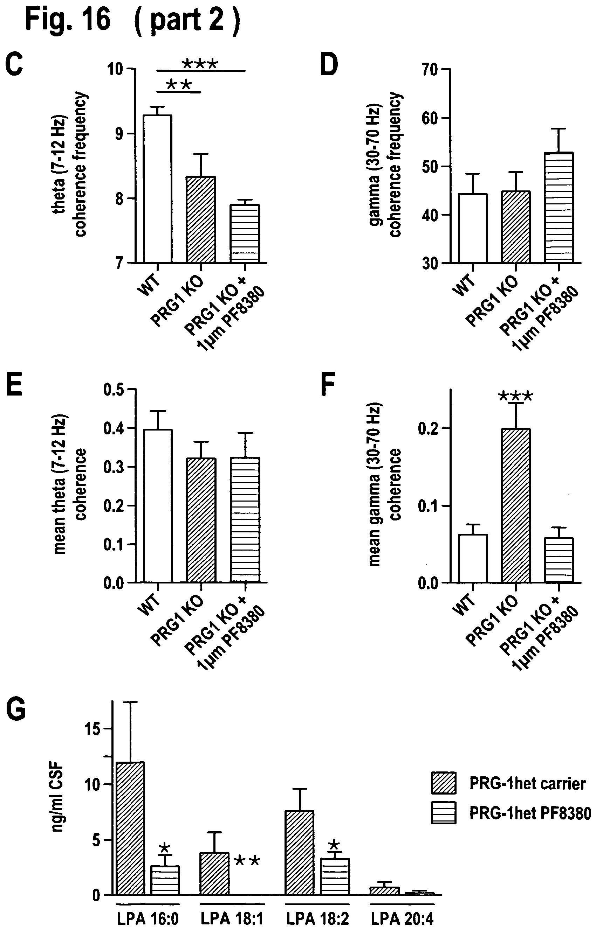

Based on this, the inventors studied PPI of the startle response in PRG-1^{+/-} (het) and wild type (wt) mice finding that PRG-1^{+/-} animals showed a significant decrease of PPI in comparison to wt littermates (Fig. 5C). To test whether pharmacological treatment is able to revert these behavioral abnormalities indicative for mental disorders, the inventors tested an *in-vivo* inhibitor of the LPA-synthetizing enzyme autotaxin (PF8380) which has a nanomolar potency and can be applied orally or by injection to the periphery (Gierse et al., 2010). Intraperitonal injection of PF8380 is able to significantly diminishe LPA levels as shown exemplarily in the cerebrospinal fluid of PRG-1 Ko mice (Figure 16G). Interstingly, PF8380 treatment effectively restored the observed PPI deficit in freely moving PRG-1^{+/-} mice to wild-type levels (Fig. 5C).

### Pharmacological intervention rescues endophenotype of psychiatric disorders in an animal model induced by a human PRG-1 variant (PRG-1^{R346T} knock-in)

In order to investigate whether the described human mutation indeed results in a endophenotype for psychiatric disorders, the inventors produced an knock-in mouse line where the PRG-1^{R346T} mutated gene was introduced in place of the PRG-1 wild-type gene. Since only monoallelic PRG-1^{R346T} mutation carriers were detected, PRG-1^{R346T+/-} animals were used for the study, which correspond to the human in-vivo situation. As shown in Figure 6, monoallelic carriers of this human mutation displayed a significantly impaired PPI, which is as described above, an endophenotype for psychiatric disorders. However, application of the autotaxin inhibitor PF8380 rescued reduced the PPI in PRG-1^{R346T+/-} animals to WT levels.

### PRG-1^{R345T}: a human SNP associated with an endophenotype for central nervous system disorders

Analysis of PRG-1 expression in the human cortex revealed strong neuronal PRG-1 signals and a clear expression in layer IV neurons (Fig 7A). Using specific markers like GAD67, parvalbumin and calretinin the inventors found specific PRG-1 expression in glutamatergic neurons but not in interneurons. Further subcellular analysis revealed dendritic co-localization of PRG-1 with the postsynaptic density marker PSD95 (Fig. 7B) confirming specific PRG-1 postsynaptic expression in glutamatergic synapses of excitatory neurons as described in the rodent brain (Trimbuch et al., 2009).

To prove for the relevance of PRG-1 and its loss-of-function SNP in humans, the inventors analyzed a well-characterized sample of 1811 healthy adult German subjects (Brinkmeyer et al., 2011; Lindenberg et al., 2011; Quednow et al., 2012) where the inventors detected the PRG-1^{R345T} (mutPRG-1) variant in at a heterozygous frequency of 0.66% (12 carriers). EEG-analysis of PRG-1^{+/mut} carriers revealed alterations of the P50 event-related potential (ERP) elicited by an auditory double-click paradigm (stimulus 1=S1, stimulus 2=S2), a measure of pre-attentive stimulus processing / sensory gating and an endophenotype for mental disorders (Javitt et al., 2008; Turetsky et al., 2007). PRG-1^{+/mut}carriers showed larger P50 amplitudes in response to the second stimulus (S2), a finding pointing to an excitation-inhibition imbalance and deficient "gating-out" of redundant information (Chang et al., 2011) (Fig. 7C). S1 reduction and S2 increase/diminished suppression may both contribute to sensory gating abnormalities in mental disorders but have been considered to reflect distinct/partially overlapping processes with a non-identical genetic architecture (Chang et al., 2011; Javitt et al., 2008). Since PRG-1 is expressed in human excitatory neurons in the somatosensory cortex (Fig. 7A,B), and PRG-1 deficient mice (PRG-1^{-/-}) display hippocampal hyperexcitability (Trimbuch et al., 2009), the electrophysiological phenotype of human PRG-1^{+/mut} carriers suggested a functional alteration of mutPRG-1. This apparently leads to an increased synaptic glutamate release at excitatory neurons thus inducing a shift in the E/I-balance towards excitation (Yizhar et al., 2011) and an altered function of cortical networks as observed in the pathophysiology of mental disorders (Belforte et al., 2010; Coyle, 2006; Javitt et al., 2011).

### ATX inhibition restores electrophysiological and behavioral consequences of cortical hyperexcitability in an animal model for schizophrenia induced by ketamine application

The inventors next analyzed whether ATX-inhibition is able to revert cortical hyperexcitability in a classical animal model for schizophrenia induced by ketamine application (Schobel SA et al. 2013; Frohlich J and JD Van Horn 2014), which does not rely on an altered synaptic phospholipid signaling but relies on neuronal hyperexcitability due to an increased glutamatergic drive. This aim was to unravel whether ATX-inhibition is able to revert cortical hyperexcitability and an altered E/I balance independent of the underlying cause. As previously described (Nosyreva E et al. 2013), ketamine application inhibits spontaneous neurotransmission and induces afterwards a potentiation of synaptic responses over time (Figure 8A). The inventors therefore inhibited ATX using 10µM HA130, an ATX-inhibitor which acts faster than PF8380 (Albers et al., 2010), during the potentiation phase. Hereby it was found that ATX inhibition significantly decreased the ketamine-induced potentiation (Fig. 8A). These results were confirmed by analysis of input output curves (Figure 8B), suggesting that ATX is a potent target in reducing excitability in this animal model for schizophrenia.

In order to correlate these electrophysiological effects with behavior in these mice, the inventors first analyzed PPI after ketamine application. Hereby, it was found significantly decreased PPI levels, which were reversible to wild type levels (WT) upon ATX-inhibition (Figure 9A). Moreover, ATX-inhibition was able to significantly decrease ketamine-induced hyperlocomotion (Figure 9B), another symptom associated with psychiatric disorders and related to increased glutamatergic input, in an open field environment. In sum, ATX-inhibition has the general potential to normalize E/I balance in psychiatric disorders associated with increased glutamatergic input and opens a new therapeutic avenue towards treatment of psychiatric disorders.

### Pharmacological intervention reduces the infarct size in a mouse model of stroke (not part of the present invention)

Neuronal hyperexcitability is a critical issue in pathophysiological states as present during ischemic stroke. Here, the so-called penumbra, a hypoxic brain region surrounding the infarct core contains viable brain tissue which has the potential to be rescued. However, hypoxia-mediated neuronal hyperexcitability leading to calcium influx and subsequent cell death dramatically jeopardizes survival of neuronal tissue in the penumbra (Lo EH 2008). Insofar, measures to reduce neuronal hyperexcitability and to induce neuroprotection in the penumbra are critically needed. As can be seen in Figure 10A, the infarct size in a mouse model of stroke was significantly reduced already after 1 day as well as after 3 days by administration of PF8380. In order to prove the effect of reduced neuronal hyperexcitability, the inventors analyzed PRG-1^{+/-} mice, which as described above, have an altered E/I balance and show neuronal hyperexcitability. In line with previous results, PF8380-treatment greatly reduced infarct volume in these mice (Figure 10B). The inventors have further assessed behavioral consequences of PF8380-treatment in PRG-1^{+/-} mice at 3 days after stroke and found significant improvement in treated mice (Figure 11A,B).

### Pharmacological intervention reduces post-synaptic currents in PRG-1 mutant mice

High excitatory excitatory post-synaptic currents (sPSC, mEPSC) are symptomatic for central nervous system disorders.

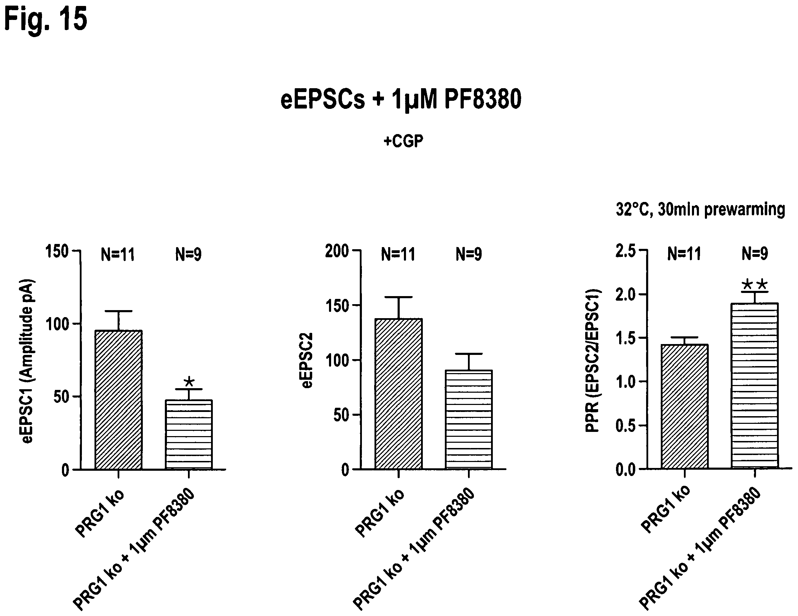

As shown in Figures 13, 14, 15 and 16, autotaxin inhibitors HA130 or PF8380 are able to reduce excitatory post-synaptic currents, which is indicative of the suitability of autotaxin inhibitors in the treatment of central nervous system disorders. Interestingly, mEPSC's of wild type mice were not affected by this intervention (Fig. 13A), suggesting that, when administered to human patients, autotaxin inhibitors might not show adverse side effects caused by overdosing.

### Pharmacological intervention rescues selectively neuronal signaling related to hyperexcitation

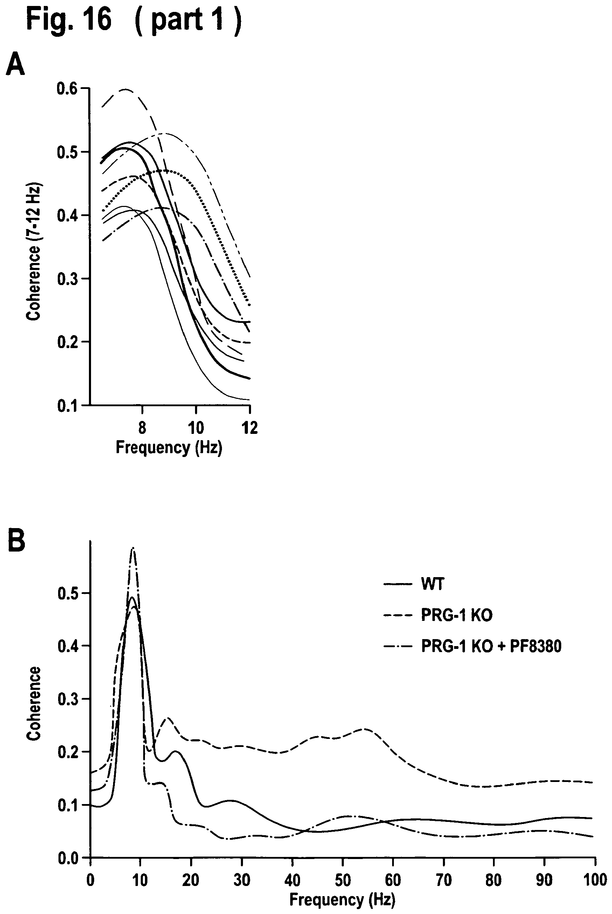

Assessment of cortico-hippocampal signaling coherence and spatial learning in PRG-1^{-/-} mice show specific impact of PF8380 on hyperexcitation-related coherence changes (mean gamma coherence, Figure 16b and f) and behavioral deficits (Figure 18b and c), not on coherence changes and behaviors derived from structural deficits in PRG-1 deficient mice (Figure 16a and c, 18a).

Pharmacological intervention selectively rescued neuronal signaling related to hyperexcitation (mean gamma coherence, Figure Figure 16b and f), however, did not affect developmental alterations described in PRG-1 deficient mice (Figure 16a and c).

As shown in Figures 16g and 17g, the autotaxin inhibitor PF 8380, which is applied by intraperitoneal injection, arrives in effective concentrations in the cerebrospinal fluid and significantly decreases the most abundant LPA-subtypes. This decrease in LPA moieties specifically resulted in rescue of altered coherence of cortico-hippocampal signaling and behaviors related to hyperexcitation. This is a selective effect, since coherence in frequencies and behaviors related to developmental (structural) changes in PRG-1 deficient mice are not changed, showing a specific action of autotaxin inhibition. Altogether, this argues for a selective therapeutic action of autotaxin inhibition in central nervous system disorders.

### ATX-inhibition suppresses obesity-related behavior

Metabolic alterations like overnight fasting induce changes in LPA subtype composition (Ino M et al. 2012). This correlates with the increased activity of ATX after overnight food deprivation, the availability of the ATX substrate LPC but also with the substrate preferences of ATX for unsaturated LPCs. In order to understand the cortical effects of altered LPA-composition upon fasting, the inventors analyzed food consumption after overnight food deprivation in control and PF8380-treated animals. While in control animals PF8380-treatment did not alter food consumption, in food deprived animals PF8380 significantly diminished food intake (Figure 19A). However, overnight food deprivation was not related to hyperlocomotion since travelled distance in an open field setting was not different between control, overnight food deprived and PF-treated overnight food deprived animals (Figure 19B).

### Methods used in the experimental Examples

### LPA Uptake Assay

HEK293 cells and HEK293 cell lines expressing wild type PRG1, PRG1^{R346T} (corresponding to the human R345T mutation), PRG1^{S346A} and PRG1^{S3460} were incubated with TF-LPA (Avanti Polar Lipids, USA, (Saunders et al., 2011)) at 1µM for 5 min at 37°C. After removing TF-LPA from the cell surface by washing the cells (3 x 5min) with 0.001%SDS in DMEM, cells were assessed by flow cytometry (FACSCantoll, Becton Dickinson, USA) and analyzed by the Flowjo software (Tree Star, USA). TF-LPA uptake was either calculated as changes of internalized TF-LPA when compared to nontransfected HEK293 cells or to PRG-1 expressing cells. In another set of experiments, cells were incubated with non-natural lipid C17-LPA (Avanti Polar Lipids, USA) instead of TF-LPA. C17-LPA and its degradation product C17MG were subsequently assessed by mass spectrometry. Primary neuronal cultures were prepared and transfected according to standard procedures.

### Quantitative phosphorylation studies

Isotopically labeled peptides (HeavyPeptide™ AQUA QuantPro) were purchased from Thermo Fisher Scientific (Waltham, MA). Following heavy isotope labeled peptides were used for assessing PRG-1 phosphorylation site occupancy: SLT DLN QDP S[R(13C6;15N4)], DAL RSL TDL NQD PS [R(13C6;15N4)], DAL R [S(PO3H2)] LTD LNQ DPS [R(13C6;15N4)], DAL T [S(PO3H2)] LTD LNQ DPS [R(13C6;15N4)] and DAL TSL TDL NQD PS [R(13C6;15N4)].

### Glycosylation studies using co-immunoprecipitation and Western blotting

For glycosylation studies co-IP was performed using Protein G agarose beads and a custom made anti-PRG-1 antibody (Trimbuch et al., 2009) according to standard procedures. Western blotting was performed using a anti-O-GlcNAc antibody (1:1000 dilution; CTD110.6, Cell Signaling, USA). For quantification, membranes were stripped and the total amount of PRG-1 was assessed using the above described anti-PRG-1 antibody.

### Pharmacological intervention

Animals were treated using the *in-vivo* potent autotaxin inhibitor PF 8380 (Gierse et al., 2010). Animals received a single i.p. injection 3 hours prior to PPI. PF8380 concentration in the cerebrospinal fluid (CSF) was measured by mass spectrometry 3 hours after injection, yielding a concentration up to 0.42 pmol / µl (with an IC50 of 0.1 pmol /µl) (Gierse et al., 2010).

### PRG-1 genotype effect on P50 event-related potential measures in humans

Study participants were investigated in the context of the "German Multicenter Study on Nicotine Dependence" (Lindenberg et al., 2011). This study was approved by the ethics committees of each study site's local university. Subjects were healthy as assessed by a standard medical examination, a drug screen and routine clinical laboratory tests. They had no current psychiatric disorder according to DSM-IV axis I as assessed by the standardized psychiatric interview (SCID-1) and were not on any medication affecting CNS function. Across study sites, the study including recordings of EEG was conducted according to the same standard operating procedure. A detailed description of stimulus presentation (auditory double click paradigm), EEG recording and signal analysis of the P50 event-related potential (ERP) has been provided in previous publications (Brinkmeyer et al, 2011; Quednow et al, 2012). The most widely-used P50 measure is the S2/S1 ratio (Turetsky et al, 2007). However, recent data suggest that individual S1 and S2 amplitudes as well as the S1-S2 difference may exhibit higher heritability (Anokhin et al, 2007) and superior test-retest reliability than the S2/S1 ratio (Fuerst et al, 2007). In view of this lack of superiority of one particular P50 measure over the others, statistical analyses of *prg-1* genotype effects on P50 ERP measures were performed on S1 and S2 amplitudes, S1-S2 difference and S2/S1 ratio.

Genotyping was performed by pyrosequencing according to manufacturer's instructions using the PSQ 96 SNP reagent kit (Qiagen, Hilden, Germany) on a PSQ HS96A instrument (Qiagen, Hilden, Germany).

### Statistical analyses

Complete datasets including P50 ERP measures and prg-1 genotype were available from six study centers for N=1811 subjects (N=12 monoallelic carriers of the *prg-1* variant vs. N=1799 wt/wt controls). There were no deviations from Hardy-Weinberg equilibrium (exact test: p=1.0). Major and minor allele frequencies (99.669% and 0.331%, respectively) were consistent with the frequencies reported previously by the NHLBI Exome Sequencing Project. Key socio-demographic sample characteristics are provided in Supplementary Table S1 (Lehrl, 1999).

To avoid confounders such as age, smoking and study site on P50 ERP measures we performed a stratified analysis of prg-1 genotype effects. For each EEG measure, three strata were determined by predicting for each individual the expected value from a regression model, incorporating age, smoking and study site, and dividing individuals into distinct, roughly equally sized strata, based on visual inspection of the histograms of predicted values. This model for stratum assignment mainly uses information from the wt/wt controls to control for confounding. In a sensitivity analysis excluding the carriers from model fitting, the results did not change. Subsequently, the standardized mean difference between prg-1 wt/wt and monoallelic R345T carriers was determined in each stratum and aggregated over the strata using inverse-variance weighting, as commonly used in fixed effect meta-analysis (Cooper, 1994). The assumption of normal distribution, required to obtain p-values corresponding to the aggregated differences, was checked by Q-Q plots. For the S1 and S2 combined effect the standardized mean difference was obtained from a linear mixed effects model incorporating a per-subject random effect. For all other measures, standardized mean differences were directly obtained from mean values and standard deviations, which were calculated separately for the carriers and wt/wt controls, i.e. the uneven size of the two groups does not skew the results. Similarly, the linear mixed effects models also are not affected by the uneven group sizes. These analyses were performed in the statistical environment R (Team, 2011) using the package "meta" (version 2.1-0).

### Immunocytochemisty on human tissue

Samples from the somatosensory cortex of a human brain were obtained from routine autopsy in accordance with local ethical committee guidelines. The subject died of non-neurological causes. Tissue was immersion-fixed in 4% PFA over 48h and snap-frozen on dry ice. Colocalization studies were performed on cryosections using a PRG-1 antibody (custom-made antibody described and characterized by (Trimbuch et al, 2009) was used in a dilution of 1:500) and antibodies against GAD67 (1:500), MAP2 (1:1000; Chemicon, Temecula USA), Parvalbumin and Calretinin (1:500; Swant, Bellinzona, Switzerland). Confocal images were taken on a Leica SP5.

### Stroke

For stroke experiments, mice were anesthetized and occlusion of the medial cerebral artery (MCAO) was performed for 30 min (assessment after 3 days) or for 1h (assessment after 1day) by introduction of an intraluminal filament as reported (Ploen et al., 2014). Treated group received PF8380 (30 mg/kg body weight) while control group received vehicle (DMSO) via i.p. application 3 hours prior to MCAO. After 1h or after 30min of MCAO, respectively, mice recovered and were perfused 24 hours (1h MCAO) or 72 hours (30min MCAO) later. Brains were extracted, snap frozen and cryosectioned using a cryotome. Mounted brain slices were Nissl-Stained which allowed to measure infarct area and to calculate total infarct volume per animal. All measurements were performed in a blinded fashion. Data was assessed for normality and statistical analysis was performed using an one-tailed t-test.

### Statistical analysis

Statistical analyses of data (including testing for normality) were performed with GraphPad Prism 5 (La Jolla, CA, USA) or with SPSS (Chicago, IL, USA). Data are presented as mean values ± s.e.m. Significance is depicted as: * p<0.05, ** p<0.001, *** p<0.001.

### Liquid chromatography-ion mobility separation-mass spectrometry (LC-IMS-MS)

Nanoscale UPLC separation was performed with a nanoAcquity UPLC system (Waters Corporation) equipped with a PepSwift Monolithic PS-DVB, 200 µm x 5 cm analytical column (Thermo Scientific Dionex). For analysis, samples (300 nL) were directly injected onto the analytical column. Mobile phase A was water containing 0.1% v/v formic acid, while mobile phase B was ACN containing 0.1% v/v formic acid. Samples were separated at 55°C with a gradient of 1-35% mobile phase B over 20 min at a flow rate of 800 nL/min. The column was rinsed for 7 min with 99% mobile phase B, followed by a re-equilibration step at initial conditions resulting in total analysis times of 40 min.

Mass spectrometric analysis of PF-8380 was performed in positive mode ESI using a Synapt G2-S HDMS mass spectrometer (Waters Corporation). Samples were analyzed using a targeted MS-method isolating the singly charged precursor of PF-8380. For fragmentation of PF-8380, collision energy was set to 28 eV. Enhanced duty cycle (EDC) was used to increase sensitivity at m/z 301. The spectral acquisition time was 0.5 s with a 0.05 s-interscan delay. All samples and references were analyzed in three technical replicates.

### Data processing

Raw data were analyzed manually using MassLynx V4.1 Software (Waters Corporation). Extracted ion chromatograms of fragment ions at m/z 301 were used for quantitative analysis calculating the area under the curve. Concentrations of PF-8380 in serum and CSF could be estimated using a reference of PF-8380 spiked into serum at known concentrations.

### Assessment of LPA species

LPA analysis was performed with liquid chromatography-electrospray ionization-tandem mass spectrometry (LC-ESI-MS/MS). The LC-MS/MS system consisted in a hybrid triple quadrupole-ion trap QTrap 5500 mass spectrometer (AB Sciex, Germany) equipped with a Turbo-V-source operating in negative ESI mode, an Agilent 1200 binary HPLC pump, column oven (40 °C) and degasser and an HTC Pal autosampler.

Two cycles of lipid-lipid extraction of LPAs were done with 1-buthanol after adding the internal standard LPA17:0 to the sample (50 µl sample, 20 µl internal standard in 500 µl 1-butanol). The combined organic phases were dried under a gentle stream of nitrogen at 45°C and subsequently re-dissolved in 200 µl methanol.

For the chromatographic separation a C18 column (Mercury 20 x 2 mm, 3 µm, 100 Å) and precolumn were used (Phenomenex, Germany). A linear gradient was employed at 400 µl/min. Mobile phase A was water with ammonium formate 50 mM and formic acid (100:0.2, v/v) and mobile phase B acetonitrile/formic acid (100:0.1, v/v), total run time 7 min, injection volume 20 µl. The mass spectrometer operated in the negative ion mode with an electrospray voltage of -4500 V at 350°C. Multiple reaction monitoring (MRM) was used for quantification. The mass transitions were m/z 409→79 (LPA 16:0), m/z 437→153 (LPA 18:0), m/z 431→79 (LPA 18:3) and m/z 457→153 (LPA 20:4) all with a dwell time of 50 ms. All quadrupoles were working at unit resolution. Quantitation was performed with Analyst Software V1.5 (Applied Biosystems) using the internal standard method with 1/concentration² weighting. The calibration curve was linear over the range of 0.1-500 ng/ml and accuracy was >95%.

### Behavioral assessment in eight-arm radial maze and in Morris Water Maze (MWM)

The radial maze (RM) consisted of a central platform of 22 cm in diameter and eight equal arms (25x6x6cm LxBxH) as described. Before training mice were food deprived to 85-90% of food weight before habituation. Two days before training, mice were habituated in the maze for 10 min/ day and trained for one trial per day on five consecutive days. Between the days arms were randomly. The mice were tested in a random order. Mice were placed in the central platform in random orientation and must collect all eight rewards. Mice visiting arms without collecting the reward and mice visiting arms already visit before were registered as working memory errors (WME). Another parameter for spatial learning was the number of new entries in arms during the first eight visited arms. A trial was terminated when all rewards were collected or after 10min.

For MWM the escape latency, swim speed, path length, and trajectory of swimming were recorded for each mouse. First, mice were trained for 5 days in "visible platform" task, than for 6 days in "hidden platform" task (spatial training), followed by a "probe trial", which was repeated for 4 times. Mice received daily 4 trials for a maximum of 90 s with an intertrial interval of 5 min. The swim velocity (mean and max) and the immobility time (time spend not searching) were measured.

### Electrophysiological assessment of freely moving behaving animals

Electrophysiological measurements in freely moving animals were performed as described (O'Neill PK et al. 2013). Briefly, animals were kept during surgical procedures under isoflurane anesthesia and electrodes (WE3PT10.5F3, MicroProbes, Gaithersburg, MD, USA) were chronically implanted in the medial entorhinal cortex (MEC; 3.1mm, lateral to the midline, 0.2mm anterior to the transverse sinus at an angle of 6° in the anterior-to-posterior direction in the sagittal plane) and in the hippocampal CA1-region (1.5mm lateral to the midline, 1.5mm anterior-to-posterior and 1.2mm dorsal-to-ventral at an angle of 0°). The electrodes were connected to an electrode interface board (EIB-16; Neuralynx, USA) and were secured to the skull using jewelers screws and dental cement. Neural data was acquired using a 16-channel head stage and a Digital Lynx data acquisition system (Neuralynx, USA). The animal's position was monitored using two light-emitting diodes mounted on the head stage. To extract local field potentials, the neural signals were bandpass filtered between 1 and 1000 Hz and digitized at 2 kHz. Separate recordings, each lasting 10 minutes, were made while animals moved freely in their home cage in an open field (0.5m x 0.5m x 0.5m) and in a plus-maze with a length of 0.4m per arm. Recordings were repeated for two days. Analysis of neural and behavioral data was performed using custom-written MATLAB scripts (MathWorks). Recording location was verified by current application at the end of the experiment after which animals were perfused, and the brains were cut on a vibratome and Nissl stained using cresyl violet.

### Field potential analysis

Coherence of neuronal activity between the MEC and in the CA1 was computed using the multitaper method (MATLAB routines provided by K. Harris, University College London, London, UK, and G. Buzsáki, New York University, New York, NY, USA). Field potentials were divided into 500 ms segments (50 ms overlap), and the Fourier transform of each segment was computed after being multiplied by two orthogonal data tapers. Coherence was then computed by averaging the cross-spectral densities of two field potential signals across data windows and tapers and normalizing to the power spectral densities of each signal. To measure oscillatory power, the wavelet transform was used. The LFP signals were convolved with a series of Morlet wavelets with center frequencies ranging from 1 to 100 Hz and a length of three cycles. Power was calculated by taking the absolute value of the wavelet transform and averaged across time to obtain the power spectrum. For statistical analysis, coherence and power were averaged within the theta (7-12 Hz) and gamma (30-70 Hz) frequency ranges.

### References and Notes

Albers HM et al., (2010) Proceedings of the National Academy of Sciences of the United States of America 107: 7257-7262
Anokhin AP et al., (2007) Schizophrenia research 89: 312-319
Belforte JE et al., (2010) Nature neuroscience 13: 76-83
Braff et al., (2001) Psychopharmacology 156: 234-58
Brinkmeyer J et al., (2011) Addict Biol 16: 485-498
Chang WP et al., (2011) Psychophysiology 48: 980-992
Chen J, Sanberg PR, Li Y, Wang L, Lu M, Willing AE, Sanchez-Ramos J, Chopp M. 2001. Intravenous administration of human umbilical cord blood reduces behavioral deficits after stroke in rats. Stroke; a journal of cerebral circulation 32:2682-2688. Cryan JF et al., (2005) Neuroscience and biobehavioral reviews 29: 571-625
Davis M (ed) (1984) The mammalian startle response. New York, NY: Plenum Press Frohlich J, Van Horn JD. 2014. Reviewing the ketamine model for schizophrenia. Journal of psychopharmacology 28:287-302.
Fuerst DR et al., (2007) Psychophysiology 44: 620-626
Gierse J et al., (2010) The Journal of pharmacology and experimental therapeutics 334: 310-317
Goswami T et al., (2012) Proteomics 12: 2185-2189
Harrison PJ, Weinberger DR (2005) Molecular psychiatry 10: 40-68; image 5
Huttlin EL et al., (2010) Cell 143: 1174-1189
Ino M, Shimizu Y, Tanaka T, Tokumura A. 2012. Alterations of plasma levels of lysophosphatidic acid in response to fasting of rats. Biological & pharmaceutical bulletin 35:2059-2063.
Inta D et al., (2010) Neuroscience and biobehavioral reviews 34: 285-294
Javitt DC et al., (2008) Nat Rev Drug Discov 7: 68-83
Lindenberg A et al., (2011) Addict Biol 16: 638-653
Lo EH. 2008. A new penumbra: transitioning from injury into repair after stroke. Nature medicine 14:497-500.
Moolenaar WH, Perrakis A (2011) Nature reviews Molecular cell biology 12: 674-679
Munton RP et al., (2007) Molecular & cellular proteomics : MCP 6: 283-293
Nosyreva E, Szabla K, Autry AE, Ryazanov AG, Monteggia LM, Kavalali ET. 2013. Acute suppression of spontaneous neurotransmission drives synaptic potentiation. The Journal of neuroscience : the official journal of the Society for Neuroscience 33:6990-7002.
Pearson RB, Kemp BE (1991) Methods in enzymology 200: 62-81
Ploen R et al., (2014) Journal of cerebral blood flow and metabolism : official journal of the International Society of Cerebral Blood Flow and Metabolism 34:495-501.
O'Neill PK, Gordon JA, Sigurdsson T. 2013. Theta oscillations in the medial prefrontal cortex are modulated by spatial working memory and synchronize with the hippocampus through its ventral subregion. The Journal of neuroscience : the official journal of the Society for Neuroscience 33:14211-14224.
Quednow BB et al., (2012) Proceedings of the National Academy of Sciences of the United States of America 109: 62716276
Radyushkin K et al., (2009) Genes, brain, and behavior 8: 416-425
Rexach JE et al., (2008) Nature chemical biology 4: 97-106
Saunders LP et al., (2011) The Journal of biological chemistry 286: 30130-30141
Schobel SA, Chaudhury NH, Khan UA, Paniagua B, Styner MA, Asllani I, Inbar BP, Corcoran CM, Lieberman JA, Moore H, Small SA. 2013. Imaging patients with psychosis and a mouse model establishes a spreading pattern of hippocampal dysfunction and implicates glutamate as a driver. Neuron 78:81-93.
Zhang L, Schallert T, Zhang ZG, Jiang Q, Arniego P, Li Q, Lu M, Chopp M. 2002. A test for detecting long-term sensorimotor dysfunction in the mouse after focal cerebral ischemia. Journal of neuroscience methods 117:207-214.
Shen S et al., (2008) The Journal of neuroscience : the official journal of the Society for Neuroscience 28: 10893-10904
Swerdlow NR et al., (1994) Arch Gen Psychiatry 51: 139-154
Tarrant MK et al., (2012) Nature chemical biology 8: 262-269
Trimbuch T et al., (2009) Cell 138: 1222-1235
Trinidad JC et al., (2008) Molecular & cellular proteomics : MCP 7: 684-696
Turetsky BI et al., (2007) Schizophrenia bulletin 33: 69-94
van Winkel R et al., (2008) Schizophrenia bulletin 34: 1095-1105
Wisniewski JR et al., (2010) Journal of proteome research 9: 3280-3289
Yizhar O et al., (2011) Nature 477: 171-178

## Claims

1. An inhibitor of autotaxin (AT) for use in the prevention or treatment of a psychiatric disorder of a subject.

2. The inhibitor for the use of claim 1, wherein said inhibitor reduces the level of lysophosphatidic acid (LPA) in the brain of said subject.

3. The inhibitor for the use of claim 1 or 2, -wherein the psychiatric disorder is selected from the group consisting of schizophrenia, depression, anxiety disorders, susceptibility to stress and stress-related disorders, panic disorders, bipolar disorder, eating disorders and ADHD.

4. The inhibitor for the use of claim 3, wherein the eating disorder is binge-eating disorder.

5. The inhibitor for the use of claim 3, wherein the psychiatric disorder is obesity or an eating disorder leading to obesity.

6. The inhibitor for the use of any one of claims 1 to 5, wherein said subject has a PRG-1 deficiency.

7. The inhibitor for the use of claim 6, wherein said PRG-1 deficiency is due to the R345T mutation in PRG-1.

8. The inhibitor for the use of any one of claims 1 to 7, wherein the autotaxin inhibitor is 4-[3-(2,3-Dihydro-2-oxo-6-benzoxazolyl)-3-oxopropyl]-1-piperazinecarboxylic acid (3,5-dichlorophenyl)methyl ester (PF8380), or (Z)-3-((4-((3-(4-Fluorobenzyl)-2,4-dioxo-1,3-thiazolan-5-yliden)-methyl)phenoxy)methyl)benzeneboronic acid (HA130).

## Patentansprüche

1. Autotaxin (AT)-Hemmer zur Verwendung bei der Vorbeugung oder Behandlung einer psychiatrischen Störung eines Patienten.

2. Hemmer zur Verwendung nach Anspruch 1, wobei der Hemmer den Lysophosphatidsäure (LPA)-Spiegel im Gehirn des Patienten senkt.

3. Hemmer zur Verwendung nach Anspruch 1 oder 2, wobei die psychiatrische Störung ausgewählt ist aus der Gruppe, bestehend aus Schizophrenie, Depression, Angststörungen, Stressanfällligkeit und stressbedingten Störungen, Panikattacken, bipolarer Störung, Essstörungen und ADHD.

4. Hemmer zur Verwendung nach Anspruch 3, wobei die Essstörung eine Binge-Eating-Störung ist.

5. Hemmer zur Verwendung nach Anspruch 3, wobei die psychiatrische Störung Adipositas oder eine zu Adipositas führende Essstörung ist.

6. Hemmer zur Verwendung nach einem der Ansprüche 1 bis 5, wobei der Patient einen PRG-1 Mangel hat.

7. Hemmer zur Verwendung nach Anspruch 6, wobei der PRG-1 Mangel auf die R345T-Mutation bei PRG-1 zurückzuführen ist.

8. Hemmer zur Verwendung nach einem der Ansprüche 1 bis 7, wobei der Autotaxin-Hemmer 4-[3-(2,3-Dihydro-2-oxo-6-benzoxazolyl)-3-oxopropyl]-1-piperazincarbonsäure (3,5-dichlorphenyl)methylester (PF8380) oder (Z)-3-((4-((3-(4-Fluorbenzyl)-2,4-dioxo-1,3-thiazolan-5-yliden)-methyl)phenoxy)methyl)benzolboronsäure (HA130) ist.

## Revendications

1. Inhibiteur de l'autotaxine (AT) à utiliser dans la prévention ou le traitement d'un trouble psychiatrique chez un sujet.

2. Inhibiteur à utiliser selon la revendication 1, dans lequel ledit inhibiteur réduit le taux de l'acide lysophosphatidique (LPA) dans le cerveau dudit sujet.

3. Inhibiteur à utiliser selon la revendication 1 ou 2, dans lequel le trouble psychiatrique est choisi parmi le groupe constitué de schizophrénie, de dépression, de troubles d'anxiété, de susceptibilité de stress et de troubles liés au stress, de troubles de panique, de trouble bipolaire, de troubles de conduites alimentaires et de TDAH.

4. Inhibiteur à utiliser selon la revendication 3, dans lequel le trouble de conduites alimentaires est l'hyperhagie.

5. Inhibiteur à utiliser selon la revendication 3, dans lequel le trouble psychiatrique est l'obésité ou un trouble de conduites alimentaires conduisant à l'obésité.

6. Inhibiteur à utiliser selon l'une quelconque des revendications 1 à 5, dans lequel ledit sujet a une carence en PRG-1.

7. Inhibiteur à utiliser selon la revendication 6, dans lequel la carence en PRG-1 est due à la mutation R345T en PRG-1.

8. Inhibiteur à utiliser selon l'une quelconque des revendications 1 à 7, dans lequel l'inhibiteur de l'autotaxine est 4-[3-(2,3-Dihydro-2-oxo-6-benzoxazolyle)-3-oxopropyle]-acide-1-pipérazinecarboxylique (3,5-dichlorophényle)méthyl ester (PF8380), ou (Z)-3-((4-((3-(4-Fluorobenzyle)-2,4-dioxo-1,3-thiazolane-5-ylidène)-méthyl)phénoxy)méthyl)acide benzèneboronique (HA130).
